# EUROPÄISCHE PATENTSCHRIFT

(11) **EP 2 902 098 B1**
(45) Veröffentlichungstag und Bekanntmachung des Hinweises auf die Patenterteilung: **19.07.2017**
(21) Anmeldenummer: 15152807.2
(22) Anmeldetag: 28.01.2015
(51) Int. Cl.: B01F 11/00, B01F 13/00, B01F 15/02, A61B 17/88

(54) **Vorrichtung und Verfahren zum Lagern und Mischen von Knochenzement**
Device and process for storing and mixing bone cement
Dispositif et procédé de stockage et de mélange de ciment osseux

(30) Priorität: 03.02.2014 DE 102014101305
(43) Veröffentlichungstag der Anmeldung: 05.08.2015
(73) Patentinhaber: Heraeus Medical GmbH, 61273 Wehrheim (DE)
(72) Erfinder: Vogt, Sebastian Dr., 99092 Erfurt (DE); Kluge, Thomas Dr., 56179 Vallendar (DE)
(74) Vertreter: Schultheiss & Sterzel Patentanwälte PartG mbB

(56) Entgegenhaltungen:
- EP-A1- 0 882 436
- EP-A1- 2 883 603
- EP-A2- 1 264 640
- WO-A1-89/05763
- DE-A1- 2 808 230
- DE-A1- 3 421 823

## Beschreibung

Die Erfindung betrifft eine Vorrichtung zur Lagerung, Vermischung und Applikation von Polymethylmethacrylat-Knochenzement und ein Verfahren zum Herstellen eines Polymethylmethacrylat-Knochenzementteigs mit einer solchen Vorrichtung. Gegenstand der Erfindung ist somit eine Vorrichtung zur Lagerung und Vermischung von Polymethylmethacrylat-Knochenzement, der vor der Vermischung, während der Lagerung, aus einer flüssigen oder pastenförmigen ersten Komponente A und dazu separaten pulverförmigen oder pastenförmigen zweiten Komponente B besteht, sowie ein Verfahren zum Vermischen und gegebenenfalls zum Applizieren der beiden Komponenten sowie, wenn gewünscht auch einem zusätzlichen pharmazeutischen Wirkstoff.

In der Medizin werden seit Jahrzehnten Polymethylmethacrylat-Knochenzemente (PMMA-Knochenzemente) zur dauerhaften mechanischen Fixierung von Totalgelenkendoprothesen eingesetzt. Diese basieren auf Pulver-Flüssigkeits-Systemen, wobei Methylmethacrylat üblicherweise als Monomer verwendet wird. Es wurden in jüngster Zeit auch Polymethylmethacrylat-Knochenzemente vorgeschlagen, die auf der Verwendung von Zementpasten beruhen (DE 10 2007 050 762 B3, DE 10 2008 030 312 A1, DE 10 2007 052 116 A1). Bei diesen Knochenzementen werden zwei Zementpasten separat in geeigneten Kartuschen gelagert. Solche Kartuschen werden häufig auch als 2-KomponentenKartuschen (auch 2K-Kartuschen) bezeichnet. Diese enthalten jeweils neben mindestens einem Monomer und geeigneten Polymeren Bestandteile eines Redoxinitiatorsystems. Die EP 1 264 640 A2 offenbart eine Vorrichtung entsprechend dem Oberbegriff des Anspruchs 1 und ein Verfahren zum Vorbereiten einer einsatzfähigen Dichtmasse beziehungsweise Klebstoffmasse in einer Kartusche.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Komponenten der Redoxinitiatorsysteme zusammenwirken. Deshalb werden die Komponenten des Redoxinitiatorsystems in den separaten Zementpasten so angeordnet, dass diese keine radikalische Polymerisation auslösen können. Die Zementpasten sind bei geeigneter Zusammensetzung lagerstabil. Erst bei Vermischung der beiden Zementpasten zu einem Zementteig reagieren die zuvor getrennt in beiden Pasten gelagerten Komponenten des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet. Zur Vermischung der Zementpasten werden üblicherweise statische Mischer eingesetzt, die dazu an die mit den Zementpasten gefüllten Zwei-Komponentenkartuschen angebracht werden.

Beim Auspressen der beiden Zementpasten aus den Kartuschen werden die beiden Zementpasten durch einen statischen Mischer gedrückt. Der Auspress- und der Vermischungsvorgang erfolgen dadurch gleichzeitig. Zur Vermischung der Zementpasten im statischen Mischer ist eine hohe Auspresskraft notwendig, weil der Druckabfall innerhalb des statischen Mischers an den Mischelementen sehr hoch ist. Dadurch ist es notwendig, leistungsfähige pneumatische oder mechanische Auspressvorrichtungen einzusetzen, um einen Austrag und eine optimale Vermischung der Zementpasten zu erreichen. Diese pneumatischen oder auch mechanischen Auspressvorrichtungen sind technisch aufwendig und teuer. Kostengünstig sind dagegen die, bisher bei den auf Pulver-Flüssigkeits-Systemen beruhenden Polymethylmethacrylat-Knochenzementen üblichen, manuell betriebenen Auspresspistolen, welche sich für diese Zemente eignen, jedoch für die Auspressung und Vermischung von Knochenzementpasten unter Verwendung von statischen Mischern nicht ausreichend leistungsfähig sind.

Bei konventionellen Zweikomponentenkartuschen ist das Volumenverhältnis der Komponenten A zur Komponente B 1:1, 1:2 und 1:10. Je ungleicher die Volumina der mit statischen Mischern zu vermischenden Komponenten sind, umso schwieriger ist es eine homogen gemischte Knochenzement-Paste zu erzeugen. Deshalb sind sehr viele Mischwendel bei größeren Volumenverhältnissen notwendig. Je größer die Anzahl der benötigten Mischwendel ist, umso größer ist auch der Druckabfall im statischen Mischer beim Mischprozess. Es muss eine pastenförmige Komponente vorhanden sein, die zweite Komponente kann flüssig oder pulverförmig oder ebenfalls pastenförmig sein. Die Komponenten beziehungsweise Pasten müssen mit sehr großer Kraft durch den statischen Mischer gepresst werden. Bei manuell betriebenen Auspressvorrichtungen ist naturgemäß die maximal mögliche Auspresskraft beschränkt.

In der Klebstoff- und Dichtungsmittelindustrie hat sich seit Jahren das System Semkit^{®} bewährt. Dabei wird in einem Lagerbehälter eine Paste gelagert. In einem Rührstab ist eine zweite flüssige Komponente enthalten, die durch ein im Rührstab integriertes Ventil von der Paste getrennt ist. Bei Betätigen des Ventils läuft die Flüssigkeit in die Paste, die dann manuell gemischt werden kann.

Nachteilig ist jedoch an einem solchen System, dass das Ventilsystem nur für zähflüssige Medien geeignet ist. Das in pastenförmigen Polymethylmethacrylat-Knochenzementen übliche Monomer Methylmethacrylat kann mit diesem Ventil nicht dauerhaft von der Paste getrennt werden. Weiterhin werden bei diesem System Volumenschwankungen, die bei der axialen Mischbewegung des Rührstabs in nicht kompressiblen Pasten auftreten, dadurch ausgeglichen, dass einerseits die Kartuschen weich sind und sich dadurch verformen beziehungsweise expandieren können und dass anderseits die Durchführung für den Rührstab nicht vollständig dicht ist, so dass gemischte Paste austreten kann und auch Luft in geringem Umfang in die gemischte Paste gezogen werden kann. Für pastenförmige Polymethylmethacrylat-Knochenzemente sind formstabile, feste Kartuschen notwendig, weil der relativ zähe pastenförmige Polymethylmethacrylat-Knochenzementteig nur mit großen Auspresskräften aus Lagerbehältern beziehungsweise Kartuschen ausgepresst werden kann. Weiterhin ist es für Knochenzemente nicht möglich, ein Mischsystem zu verwenden, bei dem ein unerwünschter Austritt geringer Pastenmengen auftritt und bei dem die Möglichkeit besteht, dass Luft in die Paste gezogen wird. Dadurch würde einerseits die Sauberkeit im Operationssaal beeinträchtigt und andererseits wird durch Eintrag von Luft der Zementteig mechanisch geschwächt, weil Luftblasen im ausgehärteten Zement als Rissansatzstellen wirken und damit die Festigkeit des ausgehärteten Knochenzements herabsetzen. Dadurch kann das Semkit^{®}-System nicht für pastenförmige Polymethylmethacrylat-Knochenzemente eingesetzt werden.

Die Aufgabe der Erfindung besteht also darin, die Nachteile des Stands der Technik zu überwinden. Insbesondere soll eine einfache und kostengünstige Vorrichtung zur Lagerung und Vermischung von Polymethylmethacrylat-Knochenzement entwickelt werden, mit der zumindest eine Polymethylmethacrylat-Knochenzementkomponente unter Luftausschluss gelagert werden kann, wobei nach Vermischung der Zementkomponenten ein Austragen des Zementteigs mit üblichen, preiswerten, manuell betriebenen Zementierpistolen möglich sein soll. Dabei soll die Hauptkomponente des Polymethylmethacrylat-Knochenzements eine Zementpaste sein und die zweite Komponente kann pastenförmig sein oder bevorzugt als Pulver vorliegen. Es darf beim Vermischen nicht zum ungewollten Austritt von Zementteig und auch nicht zum Ansaugen von Luft in den Zementteig infolge von Volumenschwankungen beim Mischvorgang kommen. Die Vorrichtung soll auch geeignet sein, bei einem Volumenverhältnis der Pasten von 1:10 bis 1:30 eine sichere Vermischung der beiden Komponenten zu gewährleisten, damit ein homogener Zementteig erhalten wird. Die beiden Komponenten des Knochenzements sollen getrennt gelagert werden können und durch Betätigung einer Verschlussvorrichtung sicher zusammengeführt werden können.

Es soll mit der vorliegenden Erfindung auch erreicht werden, dass in dem Zementteig möglichst keine Rückstände durch ein Öffnen oder Aufschneiden einer Folie, wie bei einer Verpackung oder einer Schutzfolie verbleiben können. Die Öffnung, durch die die zweite Komponente in die Hauptkomponente geleitet wird, soll eine reproduzierbare Querschnittsfläche aufweisen und sich möglichst nicht beim Mischen der Komponenten verändern. Zudem soll die Öffnung des Austragsrohrs zum Behälter, in dem die Komponenten gemischt werden, immer eine vorherbestimmte Querschnittsfläche aufweisen. Es soll möglichst nicht zu einer Beeinträchtigung dieser Querschnitte kommen können.

Eine weitere Aufgabe der Erfindung besteht darin, dass nach erfolgter Mischung der Zementkomponenten und erfolgter Öffnung des Verschlusses das Austragsrohr der Vorrichtung für den Zementteig sicher durchgängig sein muss, wobei die Öffnung des Austragsrohrs gegen ein Blockieren durch den geöffneten Verschluss während des Auspressens des Zementteigs gesichert sein muss. Weiterhin soll ein Verfahren zum Vermischen von pastenförmigen Polymethylmethacrylat-Knochenzementen unter Verwendung der zu entwickelnden Vorrichtung bereitgestellt werden.

Die Aufgaben der Erfindung werden gelöst durch eine Vorrichtung zur Lagerung, Vermischung und Applikation von Polymethylmethacrylat-Knochenzement aufweisend einen ersten Behälter für eine erste pastenförmige Komponente des Knochenzements, einen im ersten Behälter verschiebbar angeordneten Austragskolben zum Auspressen des Inhalts des ersten Behälters durch ein dem Austragskolben gegenüberliegendes Austragsrohr, wobei das Austragsrohr drehbar und in Längsrichtung verschiebbar durch eine Durchführung in einer dem Austragskolben gegenüberliegenden Seite des ersten Behälters angeordnet ist, und eine Mischeinrichtung zum Durchmischen des Inhalts des ersten Behälters, wobei die Mischeinrichtung im ersten Behälter angeordnet ist und an dem Austragsrohr befestigt ist, so dass die Mischeinrichtung im ersten Behälter durch Bewegen des Austragsrohrs zum Durchmischen des Inhalts des ersten Behälters bewegbar ist, wobei zumindest ein zweiter Behälter für zumindest eine zweite Komponente des Knochenzements an dem ersten Behälter angeordnet ist, wobei der Innenraum des zumindest einen zweiten Behälters gegen den Innenraum des ersten Behälters durch einen zu öffnenden Verschluss verschlossen ist und der zumindest eine zweite Behälter auf der dem Verschluss gegenüberliegenden Seite durch einen Dosierkolben begrenzt ist und wobei zumindest eine Begrenzungsfläche des ersten Behälters durch ein bewegliches Volumenausgleichselement gebildet ist, wobei der zumindest eine Dosierkolben mindestens ein gerichtetes Rastelement besitzt, das derart in die mindestens eine Gegenrastung an der Außenseite des zweiten Behälters greifen kann, dass nach erfolgtem Einrasten eine Rückwärtsbewegung des Dosierkolbens aus dem oder den zweiten Behältern hinaus nicht möglich ist.

Die Komponenten für den Knochenzement können erfindungsgemäß bereits in dem ersten Behälter und in dem zumindest einen zweiten Behälter enthalten sein.

Die erste Komponente ist vorzugsweise luftfrei. Die zweite Komponente ist bevorzugt pulverförmig oder pastenförmig, besonders bevorzugt eine selbststerilisierende Paste. Selbststerilisierende Pasten können beispielsweise Wasserstoffperoxid enthalten. Bevorzugt ist in einem oder mehreren der zumindest einen zweiten Behälter eine selbststerilisierende Paste enthalten, wie sie in der EP 2 596 812 A1 beschrieben ist.

Grundsätzlich reicht es zur Realisierung des Erfindungsgedankens aus, wenn das Austragsrohr bezogen auf die Wirkverbindung gegenüberliegend zum Austragskolben angeordnet ist. Eine exakte geometrische Gegenüberstellung ist nicht erforderlich. Das Gleiche gilt für den Verschluss und den Dosierkolben des zumindest einen zweiten Behälters. Auch hier gilt, dass die gegenüberliegende Seite durch die Vermittlung des Drucks gegeben ist und daher keine geometrische gegenüberliegende Positionierung des Dosierkolbens und des Verschlusses zur Realisierung des Erfindungsgedankens notwendig ist.

Ein Volumenausgleichselement kann bevorzugt durch einen oder zwei axial im zylindrischen Innenraum des ersten Behälters bewegliche Zylinder realisiert sein. Alternativ oder zusätzlich kann ein Volumenausgleichselement auch durch eine flexible verformbare Haut oder Membran gebildet sein.

Gemäß einer bevorzugten Weiterbildung der Erfindung kann auch vorgesehen sein, dass der Verschluss des zumindest einen zweiten Behälters durch Ausüben eines Drucks auf den Dosierkolben dieses zweiten Behälters zu öffnen ist, so dass dann der erste Behälter mit diesem zweiten Behälter verbunden ist.

Hierdurch ist der zweite Behälter beziehungsweise sind die zweiten Behälter zum ersten Behälter von außen leicht zu öffnen. Gleichzeitig kann durch den gleichen Druck, der den Verschluss beziehungsweise der die Verschlüsse öffnet, auch der Inhalt des zweiten Behälters beziehungsweise die Inhalte der zweiten Behälter in den ersten Behälter überführt werden. Dadurch ist nur eine einzige Bedienung der Dosierkolben oder des Dosierkolbens notwendig, um sowohl den Verschluss oder die Verschlüsse zu öffnen und den Inhalt des zweiten Behälters oder die Inhalte der zweiten Behälter in den ersten Behälter auszutragen.

Es kann erfindungsgemäß bevorzugt auch vorgesehen sein, dass der Verschluss ein Verschlussstopfen oder eine Verschlusskappe ist, der im geschlossenen Zustand in einer Öffnung in einer Trennwand zwischen dem ersten Behälter und dem zumindest einen zweiten Behälters steckt und diese Öffnung verschließt.

Gemäß einer Weiterbildung der Erfindung kann vorgesehen sein, dass die Mischeinrichtung zumindest zwei Mischflügel aufweist, die an dem ins Innere des ersten Behälters weisenden Ende des Austragsrohrs angeordnet sind und die sich radial von dem Austragsrohr nach außen in den ersten Behälter hinein erstrecken.

Mit derart geformten Mischflügeln kann eine vollständige Durchmischung des gesamten Inhalts des ersten Behälters sichergestellt werden.

Dabei kann vorgesehen sein, dass die axiale Höhe der Mischflügel größer ist als der maximale Außendurchmesser des Verschlusses, wobei der Hohlraum zwischen den Mischflügeln zumindest zur Aufnahme des Verschlusses oder der Verschlüsse ausreicht.

Hierdurch wird sichergestellt, dass die Verschlüsse oder dass der Verschluss das Herausziehen des Austragsrohrs nicht dadurch blockieren, dass sie zwischen den Mischflügeln und der Vorderseite des Innenraums des ersten Behälters, in dem die Durchführung für das Austragsrohr enthalten ist, stecken und ein weiteres Herausziehen des Austragsrohrs aus dem ersten Behälter verhindern.

Gemäß einer bevorzugten Ausgestaltung der erfindungsgemäßen Vorrichtung kann vorgesehen sein, dass die Vorrichtung zwei zweite Behälter aufweist, die beide auf zwei gegenüberliegenden Seiten durch jeweils einen Verschluss und jeweils einen Dosierkolben zum Austragen des Inhalts der zweiten Behälter begrenzt sind.

Mit den zwei Behältern können eine dritte Komponente und/oder ein zusätzlicher pharmazeutischer Wirkstoff dem Zementgemisch zugeführt werden. Bevorzugt kann erfindungsgemäß auch vorgesehen sein, dass über eine Einfüllhilfe, wie beispielsweise und erfindungsgemäß bevorzugt einen Dosiertrichter, eine dritte Komponente, bevorzugt ein Antibiotikum oder ein Antibiotika-Gemisch in einen zweiten Behälter eingefüllt wird oder einfüllbar ist, wobei die Einfüllhilfe einen, zu dem vom ersten Behälter weg weisenden Ende des zweiten Behälters passenden Einfüllstutzen aufweist. Die Vorrichtung kann erfindungsgemäß also eine solche Einfüllhilfe aufweisen, die anstatt eines Dosierkolbens in das dann offene Ende des zweiten Behälters oder eines der zweiten Behälter anzuordnen ist.

Es wird ferner vorgeschlagen, dass der zumindest eine Verschluss über eine verformbare Verbindung, insbesondere einen flexiblen Steg, mit einem Ring verbunden ist, wobei der Ring im Inneren des ersten Behälters beweglich um das Austragsrohr herum angeordnet ist, so dass die Verbindung mit dem Ring axial auf dem Austragsrohr bewegbar ist, oder der Ring an einer Führungshülse befestigt ist, die in der Durchführung für das Austragsrohr des ersten Behälters angeordnet ist und die das Austragsrohr führt.

Bevorzugt kann der Ring erfindungsgemäß selbst als Hülse ausgeführt sein. Durch den Ring kann verhindert werden, dass der Verschluss oder die Verschlüsse lose in dem ersten Behälter beweglich sind und ein Durchmischen, die Beweglichkeit der Mischeinrichtung und/oder Auspressen des Zementteigs behindern. Insbesondere können sich die Verschlüsse nicht über die Mündungsöffnung in das Austragsrohr legen und dadurch den Fluss des gemischten Zementteigs behindern.

Alternativ kann der Verschluss oder können die Verschlüsse auch an der Mischeinrichtung befestigt sein, so dass der Verschluss oder die Verschlüsse und damit der zweite Behälter oder die zweiten Behälter auch durch Einschieben des Austragsrohrs in den ersten Behälter geöffnet werden. Um eine ungewollte Öffnung des zumindest einen zweiten Behälters zu verhindern, kann vorgesehen sein, dass das Austragsrohr mit einer Verriegelungsvorrichtung gegen den zumindest einen zweiten Behälter lösbar fixiert werden kann oder fixiert ist.

Zum Verhindern eines ungewollten Austritts von Knochenzement oder der Ausgangskomponenten kann vorgesehen sein, dass in dem Austragsrohr ein axial beweglicher Kern angeordnet ist, der das Austragsrohr an dem ins Innere des ersten Behälters weisenden Ende verschließt, wobei vorzugsweise ein umlaufender Dichtungsring an dem Kern angeordnet ist, der den Kern gegen die Innenwandung des Austragsrohrs abdichtet.

Hierdurch kann sichergestellt werden, dass kein unfertiger und ungemischter Zementteig beim Mischen oder beim Einfüllen der Komponente(n) aus dem wenigstens einen zweiten Behälter in das Austragsrohr gelangt oder sogar aus dem Austragsrohr austritt.

Mit der Erfindung wird auch vorgeschlagen, dass bei geöffnetem Verschluss der Inhalt des zweiten Behälters oder die Inhalte der zweiten Behälter mit Hilfe des Dosierkolbens in den ersten Behälter überführbar ist oder sind und dann die Inhalte des ersten Behälters und des zweiten Behälters oder der zweiten Behälter im ersten Behälter miteinander mit der Mischeinrichtung mischbar sind.

Hierdurch wird eine einfache Bedienbarkeit der Vorrichtung sichergestellt.

Zur Vereinfachung der Bedienung erfindungsgemäßer Vorrichtungen kann auch vorgesehen sein, dass der Austragskolben gegen den ersten Behälter arretierbar ist oder arretiert ist, bevorzugt an dem dem Austragsrohr gegenüberliegenden Ende des ersten Behälters arretierbar ist oder arretiert ist.

Hierdurch wird eine ungewollte störende Bewegung des Austragskolbens bei der Befüllung des ersten Behälters und bei der Sterilisierung des ersten Behälters verhindert.

Es kann erfindungsgemäß besonders bevorzugt vorgesehen sein, dass der erste Behälter einen zylindrischen Innenraum aufweist und der Austragskolben in dem Innenraum des ersten Behälters eine Passform entsprechend der Grundfläche des zylindrischen Innenraums hat.

Die zylindrische Form ist die einfachste, mit der sich der erste Behälter und damit die Vorrichtung realisieren lassen. Unter einem zylindrischen Innenraum ist geometrisch ein allgemeiner Zylinder mit einer beliebigen Grundfläche zu verstehen, also nicht nur einer, mit einer kreisförmigen Grundfläche. Der Innenraum kann also ein gerader Zylinder mit beliebiger Grundfläche sein, das heißt auch mit nicht kreisförmiger oder runder Grundfläche. Erfindungsgemäß wird aber ein zylindrischer Innenraum mit kreisrunder Grundfläche bevorzugt. Diese Geometrie macht alle Bereiche des ersten Behälters für die Mischeinrichtung besonders gut erreichbar. Der Austragskolben ist dann ebenfalls zylindrisch und liegt vorzugsweise über eine Dichtung an den Wandungen des zylindrischen Innenraums des ersten Behälters an. Besonders bevorzugt ist an der dem Innenraum zugewandten Seite des Austragskolbens ein Abstreifer angeordnet, mit dem verhindert wird, dass die gemischte Knochenzementpaste beim Vortreiben des Austragskolbens am Austragskolben vorbei gedrückt wird und auf der Rückseite der Vorrichtung austreten kann. Die Mischeinrichtung hat, bei der bevorzugten kreisrunden Zylindergeometrie des ersten Behälters, Mischflügel, die gleich oder bevorzugt etwas kleiner (beispielsweise 0,1 mm kleiner) als der Innendurchmesser des zylindrischen Innenraums sind.

Die zylindrische Geometrie mit kreisrunder Grundfläche ist die einfachste zum Zweck des Aufbaus der Vorrichtung. Es ist besonders bevorzugt, wenn auch die Außenfläche des ersten Behälters entsprechend zylindrisch ist und zu wenigstens 90% die Wandung des Behälters über eine gleichmäßige Dicke verfügt. Dann kann der erste Behälter seitlich im Wesentlichen als einfaches Rohr aufgebaut werden.

Es kann ferner auch vorgesehen sein, dass der zweite Behälter oder die zweiten Behälter einen zylindrischen Innenraum aufweist oder aufweisen und der Dosierkolben in dem Innenraum des zweiten Behälters oder der zweiten Behälter eine Passform entsprechend der Grundfläche des zylindrischen Innenraums oder die Dosierkolben eine Passform entsprechend der Grundfläche der jeweiligen zylindrischen Innenräume aufweisen.

Diese Symmetrie hat die zur Symmetrie des ersten Behälters genannten Vorteile.

Eine ganz besonders bevorzugte Ausführungsform der vorliegenden Erfindung kann dadurch realisiert werden, dass das Volumenausgleichselement durch den Austragskolben realisiert ist, wobei der Austragskolben zweiteilig durch einen vorderen Austragskolben und einen Begrenzungskolben aufgebaut ist, die gegeneinander beweglich im ersten Behälter gelagert sind, und die Bewegung des vorderen Austragskolbens aus dem ersten Behälter heraus durch den Begrenzungskolben begrenzt ist, wobei der Begrenzungskolben in dem ersten Behälter arretierbar ist, wobei bevorzugt der Begrenzungskolben eine von außen lösbare Rastung aufweist, die in eine Gegenrastung am ersten Behälter greift.

Da der Austragskolben ohnehin beweglich in dem ersten Behälter angeordnet sein muss, kann dieser auch als bewegliches Volumenausgleichselement verwendet werden. Hierdurch kann auf ein separates zusätzliches bewegliches Teil verzichtet werden, wodurch der Kostenaufwand zur Herstellung der Vorrichtung niedrig gehalten werden kann.

Dabei kann vorgesehen sein, dass in dem Begrenzungskolben eine Gasdurchlassöffnung vorgesehen ist und/oder zwischen dem Begrenzungskolben und dem ersten Behälter eine Gasdurchlassöffnung vorgesehen ist, die zum Auslassen und Einfüllen eines Gases zwischen den vorderen Austragskolben und den Begrenzungskolben geeignet ist oder sind.

Hierdurch kann sichergestellt werden, dass das Gas zwischen dem vorderen Austragskolben und dem Begrenzungskolben evakuiert werden kann und der Zwischenraum mit einem sterilisierenden Gas sterilisiert werden kann. Zudem kann auch ein Druckgas in den Zwischenraum eingespeist werden, um den vorderen Austragskolben ins Innere des ersten Behälters hinein zu drücken, um den Inhalt des ersten Behälters beziehungsweise den fertig gemischten Zementteig auszupressen.

Bei erfindungsgemäßen Vorrichtungen, bei denen der Austragskolben als Volumenausgleichselement verwendet wird, kann ferner vorgesehen sein, dass am vorderen Austragskolben ein Führungselement angeordnet ist, das in einer passenden Öffnung im Begrenzungskolben geführt wird, wobei bevorzugt das Führungselement ein zylindrischer Stift ist, der sich in eine passende zylindrische Durchführung im Begrenzungskolben erstreckt.

Durch diese Maßnahme kann erreicht werden, dass der vordere Austragskolben bei einer Bewegung nicht verkantet und dadurch eine weitere Bewegung im ersten Behälter blockiert.

Mit einer weiteren Weiterbildung der Erfindung wird vorgeschlagen, dass ein lösbares Verriegelungselement eine axiale Bewegung des vorderen Austragskolbens gegen den Begrenzungskolben im Lagerzustand der Vorrichtung blockiert.

Damit wird erreicht, dass ein Befüllen des ersten Behälters und eine Sterilisierung der Vorrichtung nicht durch eine Bewegung der beiden Teile (den vorderen Austragskolben und den Begrenzungskolben) gegeneinander erschwert werden.

Mit einer Weiterentwicklung der vorliegenden Erfindung wird vorgeschlagen, dass der vordere Austragskolben über eine elastische Feder gegen den Begrenzungskolben beweglich gelagert ist, wobei die Feder zwischen dem vorderen Austragskolben und den Begrenzungskolben angeordnet ist und den vorderen Austragskolben in den Innenraum des ersten Behälters drückt. Bevorzugt ist die Feder um das Führungselement beziehungsweise um den Stift herum angeordnet, wenn ein solches beziehungsweise ein solcher vorhanden ist.

Hierdurch wird erreicht, dass der vordere Austragskolben bei sich wiederholt änderndem Volumen des ersten Behälters, das beispielsweise von dem beim Durchmischen des Inhalts des ersten Behälters durch das wiederholte Einschieben und Herausziehen des Austragsrohrs mit der Mischeinrichtung verursacht wird, wieder in die gewünschte Position durch die elastische Feder aktiv zurückgeführt wird.

Es ist erfindungsgemäß besonders vorteilhaft, wenn vorgesehen ist, dass der Raum zwischen dem verschiebbaren Austragskolben und dem Begrenzungskolben ein Volumen hat, das mindestens so groß ist wie die Summe von erstens der Volumendifferenz zwischen dem Volumen des im ersten Behälter befindlichen Teils des Austragsrohrs bei vollständig in den ersten Behälter eingeschobenem Austragsrohr und bei maximal aus dem ersten Behälter herausgezogenem Austragsrohr plus von zweitens dem Volumen der zweiten Komponente oder dem Volumen des Innenraums des einen zweiten Behälters oder der Volumina der Innenräume der zweiten Behälter.

Durch diesen Aufbau wird sichergestellt, dass der Austragskolben als Volumenausgleichselement die volle Volumenänderung des Inhalts des ersten Behälters aufnehmen kann.

Eine weitere erfindungsgemäß bevorzugte Ausgestaltung der Erfindung kann vorsehen, dass zumindest ein Volumenausgleichselement über eine elastische Feder gegen den ersten Behälter beweglich gelagert ist, wobei die Feder das Volumenausgleichselement in Richtung des Innenraums des ersten Behälters drückt. Hierdurch wird erreicht, dass das Volumenausgleichselement bei sich wiederholt änderndem Volumen des ersten Behälters, welches beim Durchmischen des Inhalts des ersten Behälters durch das wiederholte einschieben und herausziehen des Austragsrohrs mit der Mischeinrichtung entsteht, wieder in die ursprüngliche Position durch die elastische Feder aktiv zurückgeführt wird.

Es wird auch vorgeschlagen, dass an der Außenseite des mindestens einen Dosierkolbens mindestens ein poröser, gasdurchlässiger Dichtungsring angeordnet ist, so dass ein Gasaustauch aus dem von den Innenwänden des zumindest einen zweiten Behälters, dem Verschluss und dem Dosierkolben gebildeten Raum und der umgebenden Atmosphäre möglich ist.

Alternativ oder zusätzlich kann auch vorgesehen sein, dass der Dosierkolben mindestens eine gasdurchlässige Porenscheibe besitzt, so dass ein Gasaustausch aus dem von den Innenwänden des zumindest einen zweiten Behälters, dem Verschluss und dem Dosierkolben gebildeten Raum und der umgebenden Atmosphäre möglich ist.

Hierdurch kann der Inhalt des zumindest einen zweiten Behälters evakuiert und mit einem sterilisierenden Gas sterilisiert werden.

Die vorliegende Erfindung sieht vor, dass der zumindest eine Dosierkolben mindestens ein gerichtetes Rastelement besitzt, das derart in die mindestens eine Gegenrastung an der Aussenseite des zweiten Behälters greifen kann, dass nach erfolgtem Einrasten eine Rückwärtsbewegung des Dosierkolbens aus dem oder den zweiten Behältern hinaus nicht möglich ist.

Hierdurch wird sichergestellt, dass beim Auspressen des fertiggestellten Zementgemischs mit dem Austragskolben das Zementgemisch nur durch das Austragsrohr ausgetragen wird und nicht durch den zumindest einen zweiten Behälter austritt.

Ferner wird vorgeschlagen, dass der zumindest eine zweiter Behälter für die zumindest eine zweite Komponente des Knochenzements auf der gleichen Seite wie die Durchführung für das Austragsrohr angeordnet ist, insbesondere neben der Durchführung für das Austragsrohr an dem ersten Behälter angeordnet ist.

Durch diesen Aufbau wird die Bedienung der Vorrichtung vereinfacht. Dies gilt insbesondere dann, wenn der Austragskolben als Volumenausgleichselement verwendet wird und wenn eine Schraubkappe zum Vortreiben der Dosierkolben verwendet wird, die auf ein Gewinde um das Austragsrohr oder um die Durchführung für das Austragsrohr geschraubt wird.

Mit einer Weiterentwicklung der vorliegenden Erfindung wird auch vorgeschlagen, dass in dem mindestens einen zweiten Behälter, der vorzugsweise am Kopf des ersten Behälters angeordnet ist, ein separater dritter Hohlzylinder eingesteckt ist, der eine Komponente des Knochenzements enthält und der durch einen Dosierkolben und einen Verschlussstopfen verschlossen ist. Der Verschlussstopfen ist bevorzugt über einen beweglichen Steg mit dem zweiten Hohlzylinder verbunden.

Durch diese Maßnahme wird die Herstellung der Vorrichtung vereinfacht, da der separate dritte Hohlzylinder mit einer Komponente des Knochenzements und/oder einem Zusatz für den Knochenzement gefüllt werden kann und der separate dritte Hohlzylinder nach dem Befüllen erst in die Vorrichtung also in einen der zweiten Behälter eingesetzt werden muss.

Es kann auch vorgesehen sein, dass in einem am Kopf (also Vorne) des ersten Behälters angeordneten zweiten Behälter ein separater Hohlzylinder angeordnet ist, der eine dritte Komponente des Knochenzements enthält, der durch einen Verschlusstopfen und einen Dosierkolben verschlossen ist, wobei der Verschlusstopfen mit einem beweglichen Steg mit dem ersten Hohlzylinder verbunden ist und dass in einem weiteren am Kopf des ersten Behälters angeordneten zweiten Behälter ein weiterer separater Hohlzylinder angeordnet ist, der einen pharmazeutischen Wirkstoff enthält, der durch einen Verschlusstopfen und einem Dosierkolben verschlossen ist, wobei der Verschlusstopfen mit einem beweglichem Steg mit dem zweiten Hohlzylinder verbunden ist.

Es wird mit der Erfindung ferner vorgeschlagen, dass der axial bewegliche Austragskolben ein Führungselement besitzt, das in eine Führung des Austragskolbens greift.

Dabei kann auch vorgesehen sein, dass der zweite Dosierkolben eine Führung für das Führungselement des Austragskolbens besitzt und dass der zweite Dosierkolben mindestens ein durch äußere Druckbeanspruchung oder durch manuelle Einwirkung lösbares Rastelement besitzt, das den zweiten Dosierkolben reversibel an der Innenwand des ersten Behälters fixiert.

Die Aufgaben der vorliegenden Erfindung werden auch gelöst durch ein Verfahren zum Herstellen eines Polymethylmethacrylat-Knochenzements (PMMA-Knochenzements) mit einer Vorrichtung nach einem der vorangehenden Ansprüche, gekennzeichnet durch die Schritte:
A) Bereitstellen der Vorrichtung, wobei der erste Behälter mit einer ersten flüssigen oder pastenförmigen Komponente des PMMA-Knochenzements gefüllt ist und der zumindest eine zweite Behälter mit einer zweiten, vorzugsweise pulverförmigen oder pastenförmigen Komponente des PMMA-Knochenzements gefüllt ist;
B) Öffnen des zumindest einen zweiten Behälters durch Vorschieben des Dosierkolbens und Austragen der zweiten Komponente aus dem zumindest einen zweiten Behälter in den ersten Behälter durch weiteres Vortreiben des Dosierkolbens, wobei die Volumenveränderung des Inhalts des ersten Behälters beim Eintragen der zweiten Komponente in den ersten Behälter durch eine Bewegung des zumindest einen Volumenausgleichselements ausgeglichen wird, wobei der Dosierkolben oder die Dosierkolben nach vollständigem Einschieben durch eine Rastung irreversibel gegen den zumindest einen zweiten Behälter fixiert wird oder werden; und
C) Mischen der beiden Komponenten im ersten Behälter durch Bewegen der Mischeinrichtung, wobei beim Bewegen der Mischeinrichtung das mit der Mischeinrichtung verbundene Austragsrohr in den ersten Behälter wiederholt hineingeschoben und herausgezogen wird, wobei die Volumenveränderung des Inhalts des ersten Behälters beim Mischen durch eine Bewegung des zumindest einen Volumenausgleichselements ausgeglichen wird.

Dabei kann vorgesehen sein, dass nach Schritt C) ein Kern aus dem Austragsrohr entfernt wird und danach ein Schritt D) erfolgt, bei dem der gemischte Knochenzement durch Vortreiben des Austragskolbens in dem ersten Behälter durch das Austragsrohr appliziert wird.

Mit einer Weiterbildung des erfindungsgemäßen Verfahrens wird auch vorgeschlagen, dass der Inhalt des ersten Behälters gemischt wird, indem die mit dem Austragsrohr verbunden Mischeinrichtung durch hineinbewegen und herausbewegen des Austragsrohrs in den ersten Behälter in dem ersten Behälter bewegt wird, wobei bevorzugt zusätzlich die Mischeinrichtung durch Drehen des Austragsrohrs im ersten Behälter gedreht wird.

Hierdurch ist das Verfahren besonders einfach durchzuführen, da mit dem Austragsrohr nur ein bewegliches Element bedient wird, so dass die Wahrscheinlichkeit für eine Fehlbedienung reduziert ist. Zudem ist eine einfache Durchmischung der Komponenten auch unter widrigen Bedingungen außerhalb eines ordentlichen OP-Saals leicht möglich.

Ferner kann bei erfindungsgemäßen Verfahren vorgesehen sein, dass vor Schritt A) das Innere des ersten Behälters und das Innere des zumindest einen zweiten Behälters entgast und sterilisiert wird, wobei bevorzugt dazu der Begrenzungskolben, der vordere Austragskolben und/oder das Volumenausgleichselement arretiert wird, und anschließend der erste Behälter mit einer ersten Komponente des Knochenzements und der zumindest eine zweite Behälter mit einer zweiten Komponente des Knochenzements gefüllt wird, wobei bevorzugt dabei oder danach ein Antibiotikum oder ein Antibiotikagemisch in wenigstens einen zweiten Behälter gefüllt wird.

Hierdurch wird sichergestellt, dass der Inhalt steril ist. Dadurch können Infektionen beim Patienten vermieden werden.

Gemäß einer bevorzugten Ausgestaltung des erfindungsgemäßen Verfahrens kann auch vorgesehen sein, dass nach erfolgter Vermischung das Austragsrohr in Richtung aus dem ersten Behälter heraus bewegt (beziehungsweise herausgezogen) wird, so dass die Mischeinrichtung an der vorderen Innenseite des ersten Behälters anliegt.

Es ist vorgesehen, dass der Dosierkolben oder die Dosierkolben nach vollständigem Einschieben durch eine Rastung irreversibel gegen den zumindest einen zweiten Behälter fixiert wird oder werden.

Bei erfindungsgemäßen Verfahren kann also vorgesehen sein, dass bei dem Durchführen des Verfahrens, die auftretenden Volumenänderungen in dem ersten Behälter durch das Volumenausgleichselement ausgeglichen werden.

Wenn der Austragskolben durch ein Verriegelungselement arretierbar ist, kann vorgesehen sein, dass das Verriegelungselement des Austragskolbens gelöst wird, so dass der Austragskolben axial frei beweglich ist, bevor der Inhalt des zumindest einen zweiten Behälters in den ersten Behälter überführt oder eingetragen wird.

Der Erfindung liegt die überraschende Erkenntnis zugrunde, dass es durch den erfindungsgemäßen Aufbau der Vorrichtung und das erfindungsgemäße Verfahren gelingt, auf einfache Weise eine Möglichkeit zur Mischung und Applikation eines Knochenzements bereitzustellen, dessen Einzelkomponenten ein ungleiches Mischungsverhältnis von 1:10 und mehr aufweisen. Durch das Vorsehen von leicht zu bedienenden und gut zugänglichen zweiten Behältern oder eines zweiten Behälters, in dem die geringer dosierte Komponente enthalten ist, kann diese für den Anwender ohne großen Aufwand zu der Hauptkomponente zugegeben werden. Aufgrund des Volumenausgleichselements kann dabei verhindert werden, dass die Zementpaste oder die Ausgangskomponenten beim Zusammenführen aus der Vorrichtung austreten und dadurch die Umgebung und/oder der Knochenzement kontaminiert werden. Der Aufbau kann einfach und ohne hohe Kosten gefertigt, sterilisiert und mit den Ausgangskomponenten gefüllt werden. Zudem ist es möglich durch einen Dosiertrichter dem Anwender die Möglichkeit zu geben, weitere Zusätze, wie beispielsweise eine spezielle, auf den Patienten zugeschnittene Antibiotikamischung oder andere pharmazeutisch wirksame Stoffe zuzugeben.

Ein Beispiel zum Aufbau einer erfindungsgemäßen Vorrichtung ist beispielsweise eine Vorrichtung zur Lagerung und Vermischung von Polymethylmethacrylat-Knochenzement zusammengesetzt aus
a) einem zylinderförmigen Lagerbehälter mit einem ersten Behälter, der mit der pastenförmigen Komponente A befüllt ist,
b) einem Austragsrohr, das axial durch eine Durchführung am Kopf des Austragsbehälters bewegt werden kann, wobei das Austragsrohr an der dem ersten Behälter zugewandten Seite zwei oder mehrere Mischflügel enthält,
c) einem im Austragsrohr eingesteckten Kern, der das Austragsrohr auf der dem ersten Behälter zugewandten Rohrseite reversibel abschließt,
d) mindestens einem Hohlzylinder der mit dem ersten Behälter verbunden ist, wobei der Hohlzylinder einen zweiten Behälter bildet, welcher mit der Komponente B befüllt ist,
e) einem axial nur in Richtung des ersten Behälters herausdrückbarer Verschlussstopfen, der den ersten Behälter vom zweiten Behälter abtrennt,
f) mindestens einem Dosierkolben der in dem mindestens einem Hohlzylinder axial beweglich angeordnet ist,
g) wobei der zweite Behälter im Hohlzylinder durch den Verschlussstopfen und den Dosierkolben begrenzt wird,
h) einem axial verschiebbaren Austragskolben (den vorderen Austragskolben), der den ersten Behälter begrenzt,
i) einem zweiten Kolben (den Begrenzungskolben) der hinter dem vorderen Austragskolben angeordnet ist, wobei dieser zweite Kolben durch ein durch äußere Einwirkung lösbares Rastelement an der inneren Kartuschenwand (des ersten Behälters) fixiert ist, wobei der zweite Kolben mindestens einen Gasdurchlassöffnung besitzt, welche den Raum des ersten Behälters zwischen dem axial verschiebbaren vorderen Austragskolben und der umgebenden Atmosphäre verbindet, und
j) wobei der Raum zwischen dem verschiebbaren vorderen Austragskolben und dem zweiten Begrenzungskolben ein Volumen besitzt, das mindestens so groß ist wie die Summe der Volumina der Komponente B und des Austragsrohrs bei dessen maximaler Eintauchtiefe in den ersten Behälter.

Bevorzugt sind dabei zwei Hohlzylinder mit jeweils einem Verschlussstopfen und einem axial beweglichen Dosierkolben vorgesehen.

Ein erfindungsgemäßes Verfahren kann beispielsweise dadurch realisiert werden, dass
a) zuerst das Verriegelungselement des Austragskolbens gelöst wird, so dass der Austragskolben axial frei beweglich ist,
b) dass der mindestens eine Dosierkolben in Richtung des Verschlussstopfens geschoben wird, wobei der Stopfen aus dem Hohlzylinder in den ersten Behälter tritt, in dem sich die pastenförmige Komponente A befindet, und durch weitere Bewegung des Dosierkolbens die Komponente B in den ersten Behälter in die pastenförmige Komponente A gepresst wird,
c) dass der Dosierkolben nach Erreichen der Innenseite des Kopfs des Lagerbehälters durch die Rastung irreversibel im Hohlzylinder fixiert wird,
d) dass danach durch axiale und tangentiale Bewegung des Austragsrohrs mit den Mischflügeln die Komponente B mit der pastenförmigen Komponente A zum Zementteig C vermischt wird,
e) dass nach erfolgter Vermischung das Austragsrohr in Richtung des Kopfs des Lagerbehälters bewegt wird, so dass die Mischeinrichtung an der Innenseite des Lagerbehälters (des ersten Behälters) anliegt,
f) dass danach der Kern aus dem Austragsrohr herausgezogen wird, und dass dann der Begrenzungskolben und der darüber liegende vordere Austragskolben in Richtung des Kopfs der Lagervorrichtung bewegt wird, wobei der Zementteig C aus dem Raum durch das Austragsrohr in die Umgebung ausgepresst wird, und dass
g) bei den Schritten b, c und d, die Volumenänderungen des ersten Behälters, die durch Einpressung der Komponente B und das Hineinschieben und

Herausziehen des Austragsrohrs mit den Mischflügeln verursacht werden, durch axiale Bewegungen des vorderen Austragskolbens ausgeglichen werden.

Ein weiteres erfindungsgemäßes Verfahren kann beispielsweise dadurch realisiert werden, dass
a) zuerst das Verriegelungselement des Austragskolbens gelöst wird, so dass der Austragskolben axial frei beweglich ist,
b) dass der erste Dosierkolben in Richtung des Verschlussstopfens geschoben wird, wobei der Stopfen aus dem Hohlzylinder in den ersten Behälter tritt, in dem sich die pastenförmige Komponente A befindet, und durch weitere Bewegung des ersten Dosierkolbens ein pharmazeutischer Wirkstoff in den ersten Behälter in die pastenförmige Komponente A gepresst wird,
c) dass der erste Dosierkolben nach Erreichen der Innenseite des Kopfs des Lagerbehälters durch die Rastung irreversibel im Hohlzylinder fixiert wird,
d) dass dann der zweite Dosierkolben in Richtung des Verschlussstopfens geschoben wird, wobei der Stopfen aus dem Hohlzylinder in den ersten Behälter tritt, in dem sich die pastenförmige Komponente A befindet, und durch weitere Bewegung des zweiten Dosierkolbens die Komponente B1 in den ersten Behälter in die pastenförmige Komponente A gepresst wird,
e) dass der zweite Dosierkolben nach Erreichen der Innenseite des Kopfs des Lagerbehälters durch die Rastung irreversibel im Hohlzylinder fixiert wird,
f) dass danach durch axiale und tangentiale Bewegung des Austragsrohrs mit der Mischeinrichtung die Komponenten B1 und der pharmazeutische Wirkstoff mit der pastenförmigen Komponente A zum Zementteig C vermischt werden,
g) dass nach erfolgter Vermischung das Austragsrohr in Richtung des Kopfs des Lagerbehälters bewegt wird, so dass die Mischeinrichtung an der Innenseite des Lagerbehälters anliegt,
h) dass danach der Kern aus dem Austragsrohr herausgezogen wird, und dass dann der Begrenzungskolben und der darüber liegende vordere Austragskolben in Richtung des Kopfs der Lagervorrichtung bewegt wird, wobei der Zementteig C aus dem ersten Behälter durch das Austragsrohr in die Umgebung ausgepresst wird, und
i) dass bei den Schritten b, c und d, die Volumenänderungen des ersten Behälters, die durch Einpressung des pharmazeutischen Wirkstoffs und der Komponente B1 und des Hineinschiebens und Herausziehens des Austragsrohrs mit den Mischflügeln verursacht werden, durch axiale Bewegungen des vorderen Austragskolbens ausgeglichen werden.

Im Folgenden werden weitere Ausführungsbeispiele der Erfindung anhand von acht schematisch dargestellten Figuren erläutert, ohne jedoch dabei die Erfindung zu beschränken. Dabei zeigt:
- Figur 1:: eine schematische Seitenansicht einer erfindungsgemäßen Vorrichtung mit eingesetztem Einfülltrichter;
- Figur 2:: eine um 90° gedrehte schematische Seitenansicht der Vorrichtung nach Figur 1 ;
- Figur 3:: eine schematische Querschnittansicht eines Teils einer erfindungsgemäßen Vorrichtung;
- Figur 4:: eine schematische, perspektivische Querschnittansicht der in Figur 3 gezeigten Vorrichtung;
- Figur 5:: eine schematische, perspektivische Querschnittansicht der Vorrichtung nach Figur 3 und 4, bei der ein Dosierkolben eingeschoben ist;
- Figur 6:: eine schematische, perspektivische Ansicht eines als Volumenausgleichselement ausgebildeten zweiteiligen Austragskolbens für eine erfindungsgemäße Vorrichtung;
- Figur 7:: eine schematische, Querschnittansicht eines als Volumenausgleichselement ausgebildeten zweiteiligen Austragskolbens für eine erfindungsgemäße Vorrichtung; und
- Figur 8:: eine schematische Seitenansicht einer erfindungsgemäßen Vorrichtung mit eingeschobenen Dosierkolben.

In den Figuren werden teilweise für gleiche oder ähnliche Bauteile der Einfachheit halber die gleichen Bezugszeichen verwendet. Geschnittene Flächen sind schraffiert dargestellt.

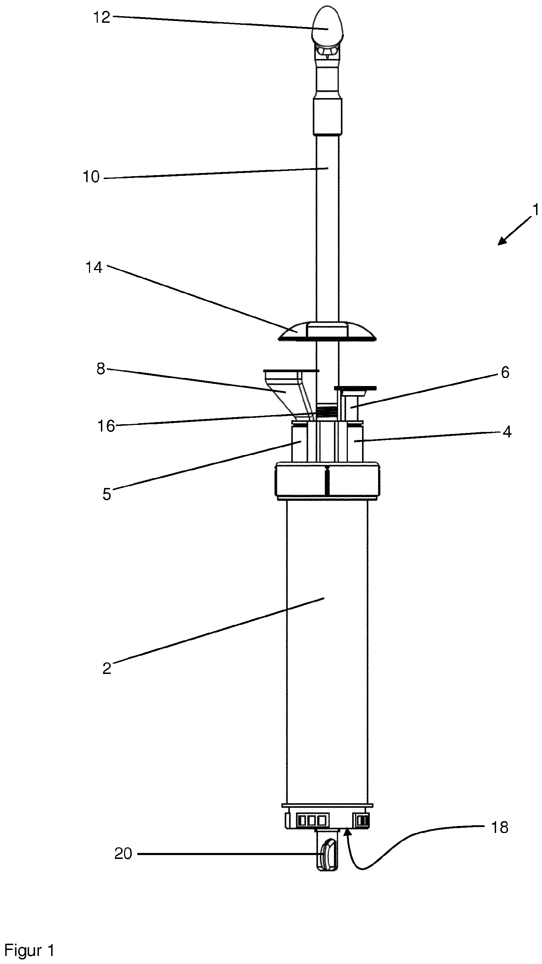

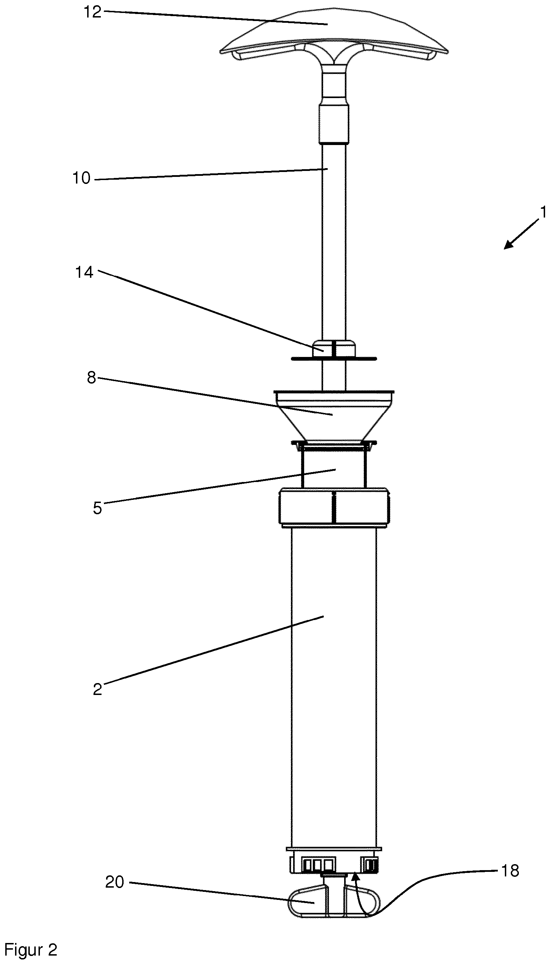

Figur 1 zeigt eine schematische Seitenansicht einer erfindungsgemäßen Vorrichtung 1 und Figur 2 eine um 90° gedrehte schematische Seitenansicht der Vorrichtung 1 nach Figur 1. Die Vorrichtung 1 ist ein Knochenzementkartuschensystem 1 zur Aufbewahrung, Lagerung, Vermischung und Applikation eines Knochenzements beziehungsweise von dessen Komponenten. Die Vorrichtung 1 und deren Teile sind im Wesentlichen aus Kunststoff, beispielsweise durch Spritzgießen hergestellt. Die Vorrichtung 1 weist einen zylindrischen ersten Behälter 2 mit einem zylindrischen Innenraum auf. In dem Innenraum des ersten Behälters 2 befindet sich eine pastöse erste Komponente beziehungsweise eine Hauptkomponente eines medizinischen Mehrkomponentenknochenzements, bevorzugt eines PMMA-Knochenzements.

An der Vorderseite des ersten Behälters 2 (in den Figuren 1 und 2 oben) sind zwei weitere (zweite) Behälter 4, 5 angeordnet, die über jeweils eine Öffnung (nicht gezeigt) mit dem ersten Behälter 2 verbunden sind, wobei in den beiden Öffnungen Verschlüsse (nicht gezeigt) angeordnet sind, die die Öffnungen verschließen und damit den ersten Behälter 2 von den zweiten Behältern 4, 5 beziehungsweise deren Innenräume trennen. Auf der zu der Öffnung gegenüberliegenden Seite des ersten zweiten Behälters 4 ist dieser zweite Behälter 4 durch einen Dosierkolben 6 verschlossen. Der erste zweite Behälter 4 ist mit einer zweiten Komponente des Knochenzements gefüllt, die pulverförmig oder pastös sein kann.

Der zweite Dosierkolben (nicht gezeigt) des anderen zweiten Behälters 5 ist herausgenommen worden und in den dadurch geöffneten anderen zweiten Behälter 5 (der zweite zweite Behälter 5) ist ein Dosiertrichter 8 eingesetzt, mit dem ein pharmazeutischer Wirkstoff (wie beispielsweise ein Antibiotikum), ein Wirkstoffgemisch, eine weitere Komponente des Knochenzements oder eine Mischung daraus in den anderen zweiten Behälter 5 eingefüllt werden kann.

Anschließend wird der zweite Dosierkolben in der gleichen Art wie der erste Dosierkolben 6 eingesetzt. Der Dosiertrichter 8 ermöglicht das Einfüllen zusätzlicher Komponenten und Wirkstoffe kurz vor der Anwendung während einer Operation (OP).

Ein Austragsrohr 10 ist durch eine gasdichte Durchführung (nicht gezeigt) des ersten Behälters 2, zwischen den beiden Öffnungen der zweiten Behälter 4, 5 beziehungsweise zwischen den beiden zweiten Behältern 4, 5, in den ersten Behälter 2 geführt. Das Austragsrohr 10, über das das fertige Knochenzementgemisch appliziert wird, ist in Längsrichtung (in den Figuren 1 und 2 von oben nach unten und umgekehrt) beweglich und drehbar in der gasdichten Durchführung gelagert. Im Inneren des ersten Behälters 2 ist an dem Austragsrohr 10 eine Mischeinrichtung (nicht gezeigt) in Form von zwei oder mehr Mischflügeln angeordnet, die sich radial vom Austragsrohr 10 weg in Richtung der Innenwände des ersten Behälters 2 bis an die Innenwände des ersten Behälters 2 oder bis dicht davor erstrecken. So kann das Austragsrohr 10 zum Durchmischen des Inhalts des ersten Behälters 2 bewegt werden. Die vordere Öffnung (in Figur 1 und 2 oben) des Austragsrohrs 10 ist mit einem Griffstück 12 verschlossen. Das Griffstück 12 ist mit einer Stange im Inneren des Austragsrohrs 10 verbunden, die sich bis zu einem Kern (nicht gezeigt) an dem ins Innere des ersten Behälters 2 weisenden Ende des Austragsrohrs 10 erstreckt und mit diesem Kern verbunden ist. Der Kern dichtet das Austragsrohr 10 ab. Das Austragsrohr 10 besteht bevorzugt aus einem transparenten Kunststoff, so dass von außen erkannt werden kann, wie weit der gemischte Knochenzementteig bereits in dem Austragsrohr 10 vorgedrungen ist, wenn das fertige Knochenzementgemisch durch das Austragsrohr 10 appliziert wird.

Der Kern lässt sich mit Hilfe des Griffstücks 12 und der Stange aus dem Austragsrohr 10 herausziehen, wodurch die Austragsöffnung geöffnet wird, die Vorrichtung 1 also zur Applikation des gemischten Knochenzementteigs einsatzbereit ist.

Um das Austragsrohr 10 herum ist eine Schraubkappe 14 mit einem Innengewinde (nicht gezeigt) angeordnet, die auf ein passendes Außengewinde 16 an der Vorderseite des ersten Behälters 2 aufgeschraubt werden kann. Wenn in beide zweiten Behälter 4, 5 die Dosierkolben 6 eingesteckt sind, können die Dosierkolben 6 durch auflegen, aufdrücken und aufschrauben der Schraubkappe 14 in die zweiten Behälter 4, 5 eingeschoben werden. Der Inhalt der beiden zweiten Behälter 4, 5 vermittelt dadurch einen Druck auf die Verschlüsse der beiden zweiten Behälter 4, 5 zum ersten Behälter 2, so dass die Verschlüsse in den Öffnungen zum ersten Behälter 2 aus diesen Öffnungen gelöst werden und in den ersten Behälter 2 hinein geschoben werden. Dadurch werden die beiden zweiten Behälter 4, 5 zum ersten Behälter 2 geöffnet.

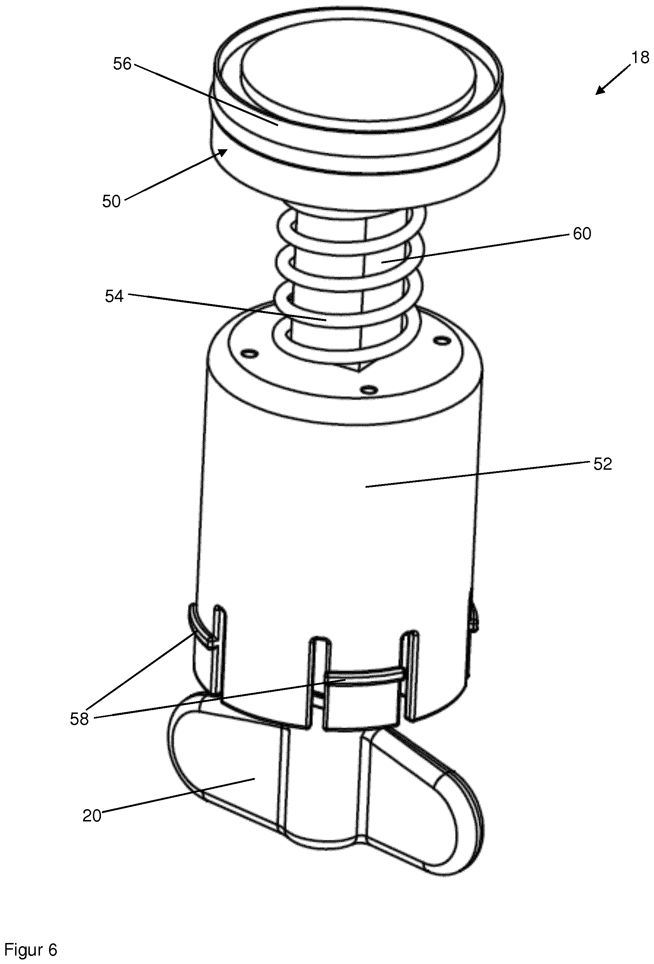

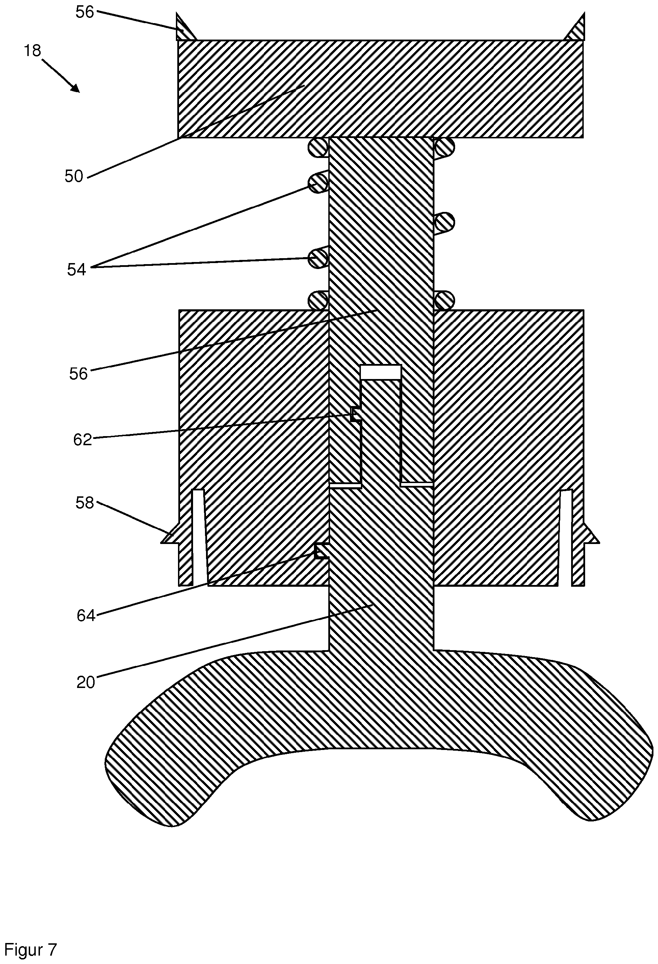

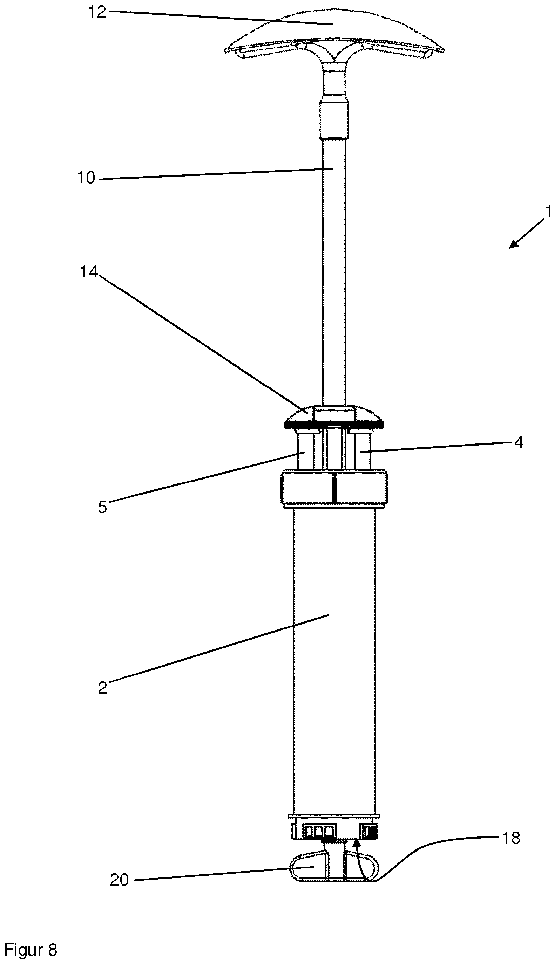

Durch weiteres Vortreiben der Dosierkolben 6 wird der Inhalt der beiden zweiten Behälter 4, 5 in den Inhalt des ersten Behälters 2 überführt. Figur 8 zeigt eine Seitenansicht der Vorrichtung 1 bei vollständig eingeschobenen Dosierkolben 6. Die dadurch entstehende Volumenzunahme im ersten Behälter 2 wird mit Hilfe eines Volumenausgleichselements aufgenommen. Als Volumenausgleichselement ist vorliegend ein bodenseitig (in Figur 1 und 2 unten) in den ersten Behälter 2 eingesetzter zweiteiliger Austragskolben 18 vorgesehen. Der erste, weiter in Innere des ersten Behälters 2 weisende vordere Austragskolben (nicht gezeigt) des Austragskolbens 18 ist in Längsrichtung der Symmetrieachse des ersten Behälters 2 beweglich gelagert. Zwischen dem bodenseitigen zweiten Teil (dem Begrenzungskolben) des Austragskolbens 18 und dem vorderen Austragskolben des Austragskolbens 18 ist eine Stahlfeder (nicht gezeigt) angeordnet, die den vorderen Austragskolben ins Innere des ersten Behälters 2 drückt. Dadurch ist der als Volumenausgleichselement ausgebildete Austragskolben 18 in der Lage sowohl Volumenänderungen aufzunehmen, die durch das Einfüllen der Inhalte der zweiten Behälter 4, 5 verursacht werden, als auch solche, die durch das Einschieben und Herausziehen des Austragsrohrs 10 in und aus dem ersten Behälter 2 erstehen. Die Funktionsweise des zweiteiligen Austragskolbens 18 wird mit Figur 6 und Figur 7 genauer erläutert.

An dem Austragskolben 18 ist eine Flügelschraube 20 zur Bedienung eines Verriegelungselements vorgesehen, mit dem durch Drehen der Flügelschraube 20 die beiden Teile des Austragskolbens 18 lösbar miteinander verriegelt werden können, so dass der vordere Austragskolben und der Begrenzungskolben nicht mehr gegeneinander axial beweglich sind. Die Verriegelung ist zum Befüllen des ersten Behälters 2 und zur Sterilisation durch Evakuieren und Befüllen mit einem sterilisierenden Gas vorteilhaft. Um eine ungewollte Bewegung des vorderen Austragskolbens zu verhindern, bleibt das Verrieglungselement bevorzugt bis unmittelbar vor dem Eintragen der Inhalte der beiden zweiten Behälter 4, 5 in den ersten Behälter 2 geschlossen. Die Flügelschraube 20 wird vor dem Austragen des fertig gemischten Zementteigs aus der Vorrichtung 1 beziehungsweise dem Austragskolben 18 herausgezogen und entfernt. Der Begrenzungskolben hat dann eine rückseitige Fläche auf der ein Stößel einer Zementpistole einen Druck ausüben kann, um beide Teile des Austragskolbens 18 vorzutreiben und dabei den Inhalt (das fertige Knochenzementgemisch) aus dem ersten Behälter 2 auszutreiben. Alternativ kann auch nur der vordere Austragskolben mit Hilfe eines Druckgases vorgetrieben werden. Dazu müssen in dem Begrenzungskolben eine oder mehrere Gasdurchführungen vorhanden sein.

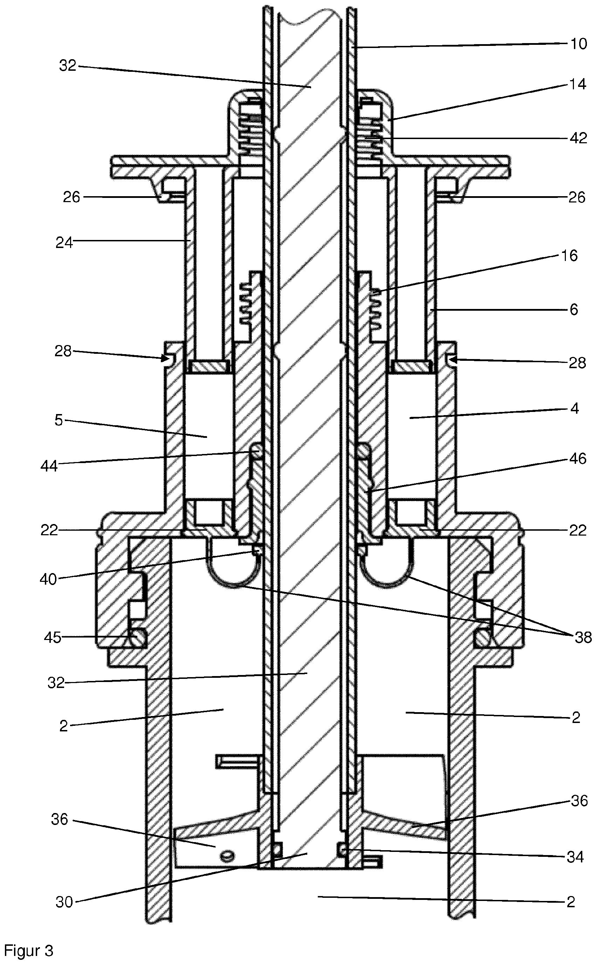

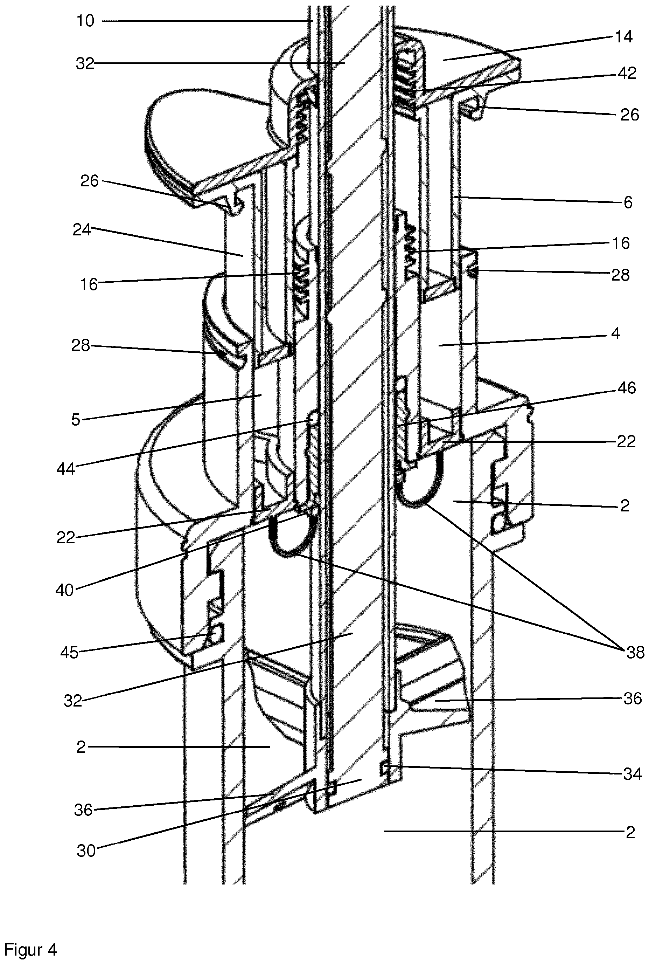

Figur 3 zeigt eine vergrößerte schematische Querschnittansicht eines Teils der erfindungsgemäßen Vorrichtung 1 und Figur 4 eine schematische, perspektivische Querschnittansicht des in Figur 3 gezeigten Teils. Die Vorrichtung 1 hat einen ersten Behälter 2 mit einem zylindrischen Innenraum. In dem zylindrischen Innenraum ist bodenseitig (in den Figuren 3 bis 5 unten) ein in Richtung der Symmetrieachse beziehungsweise Längsachse des Innenraums des ersten Behälters 2 passender, beweglicher, aber lösbar arretierbarer zylindrischer Austragskolben (in den Figuren 3 bis 5 nicht zu sehen) angeordnet.

Die Vorderseite (in den Figuren 3 bis 5 oben) des ersten Behälters 2 ist teilweise durch zwei zweite Behälter 4, 5 abgedeckt. In den Öffnungen der zweiten Behälter 4, 5 zum ersten Behälter 2 ist jeweils ein Stopfen 22 vorgesehen, die die zweiten Behälter 4, 5 vom ersten Behälter 2 trennen, beziehungsweise die deren Innenräume voneinander trennen. An der Vorderseite der beiden zweiten Behälter 4, 5 sind diese durch zwei Dosierkolben 6, 24 verschlossen. Die Außenform der Dosierkolben 6, 24 entspricht der Innenform der zweiten Behälter 4, 5 und die Dosierkolben 6, 24 sind in Längsrichtung der zweiten Behälter 4, 5 (in den Figuren 3 bis 5 von oben nach unten) verschiebbar, so dass sich die Inhalte der zweiten Behälter 4, 5 mit den Dosierkolben 6, 24 in der ersten Behälter 2 pressen lassen.

Die beiden zweiten Behälter 4, 5 und die Dosierkolben 6, 24 sind zylindrisch mit einer nierenförmigen Grundfläche, das heißt einem nierenförmigen Querschnitt senkrecht zur Zylinderachse, und sind um das Austragsrohr 10 herum benachbart angeordnet.

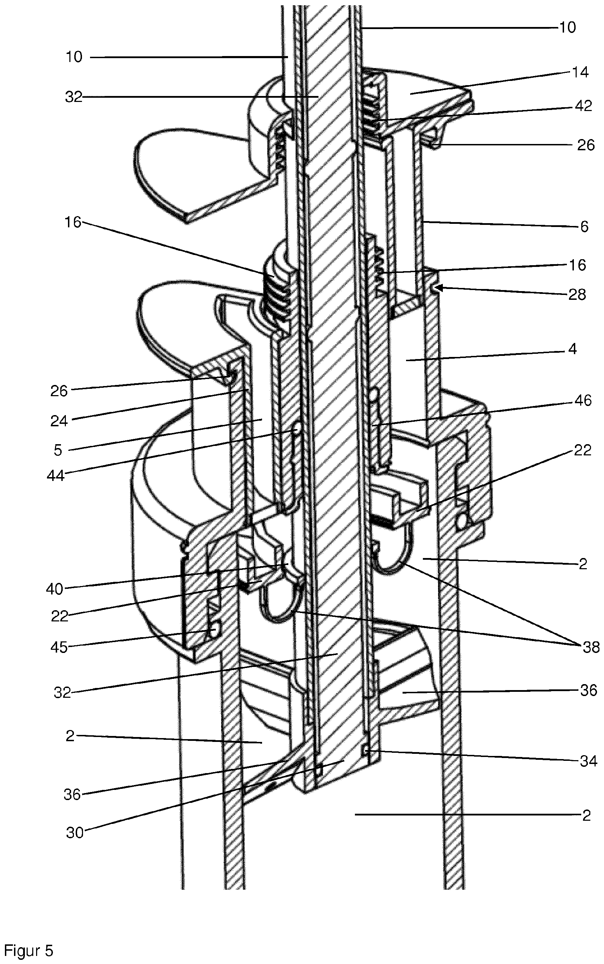

Durch das Einschieben der Dosierkolben 6, 24 werden zunächst die Stopfen 22 aus den Öffnungen zum ersten Behälter 2 herausgedrückt. Anschließend wird der Inhalt der zweiten Behälter 4, 5 in den ersten Behälter 2 gedrückt. Herzu zeigt Figur 5 eine schematische, perspektivische Querschnittansicht des Teils der Vorrichtung 1, der auch in den Figuren 3 und 4 gezeigt ist, bei der ein Dosierkolben 24 vollständig in einen der zweiten Behälter 5 eingeschoben ist. An der Oberseite der Dosierkolben 6, 24 sind Rastelemente 26 vorgesehen, die in Gegenrastungen 28 an der Außenseite der zweiten Behälter 4, 5 greifen. Sobald die Dosierkolben 6, 24 voll eingeschoben sind, rasten die Rastelemente 26 in die Gegenrastungen 28, so dass die Dosierkolben 6, 24 nicht mehr aus den zweiten Behältern 4, 5 heraus bewegt werden können. Dadurch wird sichergestellt, dass beim Auspressen des Inhalts des ersten Behälters 2 mit dem Austragskolben das fertige Knochenzementgemisch nicht in und durch die zweiten Behälter 4, 5 heraus gedrückt werden kann.

Stattdessen wird das fertige Knochenzementgemisch durch das Austragsrohr 10 ausgepresst und kann über dessen Spitze am Knochen eines Patienten während einer OP appliziert werden. Dazu muss zuvor ein Kern 30, der das Austragsrohr 10 von Innen verschließt, mit Hilfe einer mit dem Kern 30 verbundenen Stange 32 aus dem Austragsrohr 10 herausgezogen werden. Der Kern 30 ist mit einer umlaufenden Dichtung 34, wie beispielsweise einem O-Ring aus Gummi, gegen die Innenwand des Austragsrohrs 10 abgedichtet. Der Kern 30 befindet sich an dem ins Innere des ersten Behälters 2 weisen Ende des Austragsrohrs 10.

An diesem Ende des Austragsrohrs 10 sind auch Mischflügel 36 angeordnet, die sich radial vom Austragsrohr 10 weg in Richtung der Innenwände des ersten Behälters 2 erstrecken und bis auf 0,1 mm oder sogar noch dichter an diese heranreichen.

Die beiden Stopfen 22 sind über zwei Stege 38 mit einem Ring 40 verbunden, der um das Austragrohr 10 herum angeordnet ist. Mit Hilfe der Stege 38 und des Rings 40 wird dafür gesorgt, dass die Stopfen 22 sich nicht unkontrolliert im ersten Behälter 2 bewegen können und dabei die Durchmischung des Inhalts des ersten Behälters 2 mit Hilfe der Mischeinrichtung 36 verhindern oder behindern. Zudem wird mit dem Ring 40 und den Stegen 38 verhindert, dass sich die Stopfen 22 ungewollt vor die Einmündung in das Austragsrohr 10 setzen und dadurch den Fluss des fertigen Knochenzementgemischs in das Austragsrohr 10 behindern.

Vor den beiden Dosierkolben 6, 24 ist eine Schraubkappe 14 drehbar um das Austragsrohr 10 herum angeordnet. Die Schraubkappe 14 hat ein Innengewinde 42, das auf ein Außengewinde 16 vor den beiden zweiten Behälters 4, 5 aufgeschraubt werden kann, wobei dabei die Dosierkolben 6, 24 in die zweiten Behälter 4, 5 eingeschoben werden. Die Dosierkolben 6, 24 weisen gasdurchlässige Öffnungen (nicht gezeigt) auf.

Die verschiedenen Einzelteile der Vorrichtung 1 sind durch Dichtungsringe 44, 45 aus Gummi gegeneinander abgedichtet, so dass der erste Behälter 2 evakuiert werden kann und dass der Inhalt des ersten Behälters 2 bei Beaufschlagung mit einem Druck durch den Austragskolben nicht durch die Zwischenräume herausgepresst werden kann.

Das Austragsrohr 10 ist durch eine Führungshülse 46 in den ersten Behälter 2 geführt und mit einem Dichtungsring 44 abgedichtet. Der Kopf der Kartusche (des ersten Behälters 2), an dem die beiden zweiten Behälter 4, 5 ausgebildet sind, steckt nach Art eine Kappe mit einer Rastung auf dem Zylinder, der die Wandung des ersten Behälters 2 bildet, und auf der Führungshülse 46 auf. Der Kopf der Kartusche ist gegen die Zylinderwandung des ersten Behälters 2 mit einem Dichtungsring 45 abgedichtet.

Figur 6 zeigt eine schematische, perspektivische Ansicht eines als Volumenausgleichselement ausgebildeten zweiteiligen Austragskolbens 18 einer erfindungsgemäßen Vorrichtung und Figur 7 zeigt eine schematische, Querschnittansicht eines als Volumenausgleichselement ausgebildeten zweiteiligen Austragskolbens 18, wie er in den Vorrichtungen 1 nach den Figuren 1 bis 5 und 8 eingesetzt sein kann. Der vordere erste Teil (in Figur 6 oben), das heißt der vordere Austragskolben 50, des Austragskolbens 18 ist beweglich gegen den hinteren zweiten Teil, das heißt den Begrenzungskolben 52 des Austragskolbens 18 gelagert. Zwischen den beiden Teilen 50, 52 des Austragskolbens 18 ist eine elastische Feder 54 aus Stahl oder einem elastischen Kunststoff angeordnet, die den vorderen Austragskolben 50 ins Innere des ersten Behälters beziehungsweise in Figur 6 und 7 nach oben drückt.

In dem Begrenzungskolben 52 ist eine gasdurchlässige Durchführung (nicht gezeigt) vorgesehen, die ein Entweichen der Luft aus dem Zwischenraum zwischen den beiden Teilen 50, 52 ermöglicht, um ein Sterilisieren des Zwischenraums mit einem sterilisierenden Gas zu ermöglichen und gegebenenfalls um den vorderen Austragskolben 50 mit Hilfe eines Gasdruck vorzutreiben, ohne den Begrenzungskolben 52 bewegen zu müssen. An der Oberkante des vorderen Austragskolbens 50 ist eine umlaufende Abstreiflippe 56 angeordnet. Die Abstreiflippe 56 liegt an den Innenwänden des ersten Behälters 2 an, wenn der vordere Austragskolben 50 in den ersten Behälter 2 eingesetzt ist, und sorgt dafür, dass keine Knochenzementreste in dem ersten Behälter 2 zurückbleiben, beziehungsweise dass kein Knochenzement aus der Hinterseite der Vorrichtung 1 austreten kann.

Ein mit einer Flügelschraube 20 lösbares Verriegelungselement kann zur Arretierung des vorderen Austragskolbens 50 gegen den Begrenzungskolben 52 verwendet werden. Über mehrere am Außenumfang des Begrenzungskolbens 52 angeordnete und radial nach Innen biegbare Rastelemente 58, wie beispielsweise vorspringende Federn beziehungsweise Leisten, wird der Begrenzungskolben 52 mit entsprechend an den Innenwänden des ersten Behälters 2 angeordneten Gegenrastmitteln (nicht gezeigt), wie beispielsweise eine umlaufende Nut, verbunden, so dass der Begrenzungskolben 52 nicht mehr ohne weiteres aus dem ersten Behälter 2 entfernt werden kann, wenn er erst einmal eingesteckt wurde.

An dem Austragskolben 18 ist eine Flügelschraube 20 zur Bedienung des Verriegelungselements vorgesehen, wobei durch Drehen der Flügelschraube 20 die beiden Teile 50, 52 des Austragskolbens 18 lösbar miteinander verriegelt werden können, so dass der vordere Austragskolben 50 und der Begrenzungskolben 52 nicht mehr gegeneinander axial beweglich sind. Die Verriegelung ist zum Befüllen des ersten Behälters 2 und zur Sterilisation des Zwischenraums zwischen den beiden Teilen 50, 52 durch Evakuieren und Befüllen mit einem sterilisierenden Gas vorteilhaft. Um eine ungewollte Bewegung des Austragskolbens 50 gegen den Begrenzungskolben 52 zu verhindern, bleibt das Verriegelungselement bevorzugt bis unmittelbar vor dem Eintragen der Inhalte der beiden zweiten Behälter 4, 5 in den ersten Behälter 2 geschlossen.

Die Flügelschraube 20 wird vor dem Austragen des fertig gemischten Zementteigs aus der Vorrichtung beziehungsweise dem Austragskolben 18 herausgezogen und entfernt. Der Begrenzungskolben 52 hat dann eine rückseitige Fläche auf der ein Stößel einer Zementpistole einen Druck ausüben kann, um beide Teile 50, 52 des Austragskolbens 18 vorzutreiben und dabei den Inhalt (das fertige Knochenzementgemisch) aus dem ersten Behälter 2 auszutreiben. Alternativ kann auch nur der vordere Austragskolben 50 mit Hilfe eines Druckgases vorgetrieben werden. Dazu müssen in oder neben dem Begrenzungskolben 52 eine oder mehrere Gasdurchführungen vorhanden sein.

Auf der Zylinderachse des vorderen Austragskolbens 50 ist ein Führungselement 60 in Form eines zylindrischen Stifts mit quadratischer oder rechteckiger Grundfläche befestigt, der sich bis in eine Ausnehmung des Begrenzungskolbens 52 erstreckt. Das Führungselement 60 verhindert ein Verkippen und damit ein Verkannten des vorderen Austragskolbens 50.

In der Querschnittansicht nach Figur 7 ist erkennbar, wie das Verriegelungselement funktioniert und mit der Flügelschraube 20 bedient werden kann. Die Flügelschraube 20 hat einen Schaft mit zwei verschiedenen Durchmessern. Der vordere dünnere Schaft kann in eine passende dünnere Ausnehmung in dem Führungselement 60 des vorderen Austragskolbens 50 eingeschoben werden. Über einen ersten Bajonett-Verschluss 62 kann der dünnere Schaft mit dem Führungselement 60 verriegelt werden. Am dickeren Schaft ist ein zweiter Bajonett-Verschluss 64 vorgesehen, der den dickeren Schaft mit dem Begrenzungskolben 52 verriegeln kann. Dadurch kann mit Hilfe des aus den beiden Schaftteilen, den Bajonett-Verschlüssen 62, 64 und der Flügelschraube 20 gebildeten Verriegelungselements der vordere Austragskolben 50 mit dem Begrenzungskolben 52 fest verbunden werden.

Die Rastmittel 58 des Begrenzungskolbens 52 sind an biegbaren Stegen angeordnet und greifen beim Einsetzen in eine umlaufende Nut an den Innenwänden des ersten Behälters 2, wobei die Nut im Bereich der Rückseite des ersten Behälters 2 angeordnet ist. Der Begrenzungskolben 52 weist Gasdurchlässe (nicht gezeigt) auf, durch die ein Gas zwischen dem vorderen Austragskolben 50 und dem Begrenzungskolben 52 evakuiert oder eingespeist werden kann.

Durch die Stege und das Rastmittel 58 kann der Begrenzungskolben 52 manuell von außen von den Wandungen des ersten Behälters 2 gelöst werden. Dazu ist das Gegenrastmittel beziehungsweise die Nut an der Innenwand des ersten Behälters 2 derart dicht am hinteren Ende des ersten Behälters 2 angeordnet, dass der hintere Teil der Stege über den hinteren Rand des ersten Behälters 2 hinausragt.

Figur 8 zeigt eine schematische Seitenansicht einer erfindungsgemäßen Vorrichtung 1 mit eingeschobenen Dosierkolben. Die Schraubkappe 14 wurde vollständig auf das Außengewinde aufgeschraubt. Dabei wurden die Inhalte der geöffneten zweiten Behälter 4, 5 vollständig in den ersten Behälter 2 überführt.

Durch Drehen, Hineinschieben und Herausziehen des Austragsrohrs 10 wird das daran befestigte Mischelement im Inneren des ersten Behälters 2 bewegt und der Inhalt des ersten Behälters 2 durchmischt. Das Austragsrohr 10 wird nach dem Durchmischen bis zum Anschlag aus dem Innenraum des ersten Behälters 2 (in Figur 7 nach oben) herausgezogen. Dabei legen sich die losen Stopfen 22 in ausreichend groß dimensionierte Zwischenräume zwischen den Mischflügeln 36 der Mischeinrichtung 36 an. Anschließend wird der Kern 30 im Inneren des Austragsrohrs 10 mit Hilfe des Griffstücks 12 aus dem Austragsrohr 10 herausgezogen und die Vorrichtung 1 damit nach außen geöffnet.

Durch Vortreiben des Austragskolbens 18 oder des vorderen Austragskolbens 50 in den ersten Behälter 2 hinein, wird der fertig durchmischte Knochenzementteig aus dem ersten Behälter 2 über das Austragsrohr 10 ausgetragen und kann appliziert werden.

Der Aufbau der Vorrichtung 1 und ihrer Teile ist in vielen Teilen Symmetrisch um eine bezogen auf die Figuren vertikale Symmetrieachse beziehungsweise bezogen auf eine Symmetrieebene, in der sich diese Symmetrieachse befindet.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Vorrichtung / Knochenzementkartuschensystem
- 2: erster Behälter
- 4: erster zweiter Behälter
- 5: zweiter zweiter Behälter
- 6, 24: Dosierkolben
- 8: Dosiertrichter / Einfüllhilfe
- 10: Austragsrohr
- 12: Griffstück
- 14: Schraubkappe
- 16: Außengewinde
- 18: Austragskolben
- 20: Flügelschraube
- 22: Stopfen / Verschluss
- 26: Rastelement
- 28: Gegenrastmittel
- 30: Kern
- 32: Stange
- 34: Dichtung
- 36: Mischflügel / Mischeinrichtung
- 38: Steg
- 40: Ring
- 42: Innengewinde
- 44, 45: Dichtungsring
- 46: Führungshülse
- 50: Vorderer Austragskolben
- 52: Begrenzungskolben
- 54: Feder
- 56: Abstreiflippe
- 58: Rastmittel / Feder
- 60: Führungselement / Stift
- 62: Bajonett-Verschluss mit Führungselement
- 64: Bajonett-Verschluss mit Begrenzungskolben

## Patentansprüche

1. Vorrichtung (1) zur Lagerung, Vermischung und Applikation von Polymethylmethacrylat-Knochenzement aufweisend einen ersten Behälter (2) für eine erste pastenförmige Komponente des Knochenzements, einen im ersten Behälter (2) verschiebbar angeordneten Austragskolben (18) zum Auspressen des Inhalts des ersten Behälters (2) durch ein dem Austragskolben (18) gegenüberliegendes Austragsrohr (10), wobei das Austragsrohr (10) drehbar und in Längsrichtung verschiebbar durch eine Durchführung in einer dem Austragskolben (18) gegenüberliegenden Seite des ersten Behälters (2) angeordnet ist, und eine Mischeinrichtung (36) zum Durchmischen des Inhalts des ersten Behälters (2), wobei die Mischeinrichtung (36) im ersten Behälter (2) angeordnet ist und an dem Austragsrohr (10) befestigt ist, so dass die Mischeinrichtung (36) im ersten Behälter (2) durch Bewegen des Austragsrohrs (10) zum Durchmischen des Inhalts des ersten Behälters (2) bewegbar ist, wobei zumindest ein zweiter Behälter (4, 5) für zumindest eine zweite Komponente des Knochenzements an dem ersten Behälter (2) angeordnet ist, wobei der Innenraum des zumindest einen zweiten Behälters (4, 5) gegen den Innenraum des ersten Behälters (2) durch einen zu öffnenden Verschluss (22) verschlossen ist und der zumindest eine zweite Behälter (4, 5) auf der dem Verschluss (22) gegenüberliegenden Seite durch einen Dosierkolben (6, 24) begrenzt ist und wobei zumindest eine Begrenzungsfläche des ersten Behälters (2) durch ein bewegliches Volumenausgleichselement (50) gebildet ist, **dadurch gekennzeichnet, dass** der zumindest eine Dosierkolben (6, 24) mindestens ein gerichtetes Rastelement (26) besitzt, das derart in die mindestens eine Gegenrastung (28) an der Außenseite des zweiten Behälters (4, 5) greifen kann, dass nach erfolgtem Einrasten eine Rückwärtsbewegung des Dosierkolbens (6, 24) aus dem oder den zweiten Behältern (4, 5) hinaus nicht möglich ist.

2. Vorrichtung (1) nach Anspruch 1, **dadurch gekennzeichnet, dass** der Verschluss (22) des zumindest einen zweiten Behälters (4, 5) durch Ausüben eines Drucks auf den Dosierkolben (6, 24) dieses zweiten Behälters (4, 5) zu öffnen ist, so dass dann der erste Behälter (2) mit diesem zweiten Behälter (4, 5) verbunden ist.

3. Vorrichtung (1) nach Anspruch 1 oder 2, **dadurch gekennzeichnet, dass** die Mischeinrichtung (36) zumindest zwei Mischflügel (36) aufweist, die an dem ins Innere des ersten Behälters (2) weisenden Ende des Austragsrohrs (10) angeordnet sind und die sich radial von dem Austragsrohr (10) nach außen in den ersten Behälter (2) hinein erstrecken.

4. Vorrichtung (1) nach Anspruch 3, **dadurch gekennzeichnet, dass** die axiale Höhe der Mischflügel (36) größer ist als der maximale Außendurchmesser des Verschlusses (22), wobei der Hohlraum zwischen den Mischflügeln (36) zumindest zur Aufnahme des Verschlusses (22) oder der Verschlüsse (22) ausreicht.

5. Vorrichtung (1) nach einem der vorangehenden Ansprüche, **dadurch gekennzeichnet, dass**
die Vorrichtung (1) zwei zweite Behälter (4, 5) aufweist, die beide auf zwei gegenüberliegenden Seiten durch jeweils einen Verschluss (22) und jeweils einen Dosierkolben (6, 24) zum Austragen des Inhalts der zweiten Behälter (4, 5) begrenzt sind.

6. Vorrichtung (1) nach einem der vorangehenden Ansprüche, **dadurch gekennzeichnet, dass**
der zumindest eine Verschluss (22) über eine verformbare Verbindung (38), insbesondere einen flexiblen Steg (38), mit einem Ring (40) verbunden ist, wobei der Ring (40) im Inneren des ersten Behälters (2) beweglich um das Austragsrohr (10) herum angeordnet ist, so dass die Verbindung (38) mit dem Ring (40) axial auf dem Austragsrohr (10) bewegbar ist, oder der Ring (40) an einer Führungshülse (46) befestigt ist, die in der Durchführung für das Austragsrohr (10) des ersten Behälters (2) angeordnet ist und die das Austragsrohr (10) führt.

7. Vorrichtung (1) nach einem der vorangehenden Ansprüche, **dadurch gekennzeichnet, dass**
der Austragskolben (18) gegen den ersten Behälter (2) arretierbar ist oder arretiert ist, bevorzugt an dem dem Austragsrohr (10) gegenüberliegenden Ende des ersten Behälters (2) arretierbar ist oder arretiert ist.

8. Vorrichtung (1) nach einem der vorangehenden Ansprüche, **dadurch gekennzeichnet, dass**
das Volumenausgleichselement (50) durch den Austragskolben (18) realisiert ist, wobei der Austragskolben (18) zweiteilig durch einen vorderen Austragskolben (50) und einen Begrenzungskolben (52) aufgebaut ist, die gegeneinander beweglich im ersten Behälter (2) gelagert sind, und die Bewegung des vorderen Austragskolbens (50) aus dem ersten Behälter (2) heraus durch den Begrenzungskolben (52) begrenzt ist, wobei der Begrenzungskolben (52) in dem ersten Behälter (2) arretierbar ist, wobei bevorzugt der Begrenzungskolben (52) eine von außen lösbare Rastung (58) aufweist, die in eine Gegenrastung am ersten Behälter (2) greift.

9. Vorrichtung (1) nach Anspruch 8, **dadurch gekennzeichnet, dass**
in dem Begrenzungskolben (52) eine Gasdurchlassöffnung vorgesehen ist und/oder zwischen dem Begrenzungskolben (52) und dem ersten Behälter (2) eine Gasdurchlassöffnung vorgesehen ist, die zum Auslassen und Einfüllen eines Gases zwischen dem vorderen Austragskolben (50) und dem Begrenzungskolben (52) geeignet ist oder sind.

10. Vorrichtung (1) nach einem der vorangehenden Ansprüche, **dadurch gekennzeichnet, dass**
zumindest ein Volumenausgleichselement (50) über eine elastische Feder (54) gegen den ersten Behälter (2) beweglich gelagert ist, wobei die Feder (54) das Volumenausgleichselement (50) in Richtung des Innenraums des ersten Behälters (2) drückt.

11. Vorrichtung (1) nach einem der vorangehenden Ansprüche, **dadurch gekennzeichnet, dass**
an der Außenseite des mindestens einen Dosierkolbens (6, 24) mindestens ein poröser, gasdurchlässiger Dichtungsring angeordnet ist, so dass ein Gasaustauch aus dem von den Innenwänden des zumindest einen zweiten Behälters (4, 5), dem Verschluss (22) und dem Dosierkolben (6, 24) gebildeten Raum und der umgebenden Atmosphäre möglich ist.

12. Verfahren zum Herstellen eines Polymethylmethacrylat-Knochenzements mit einer Vorrichtung (1) nach einem der vorangehenden Ansprüche, mit den Schritten:
A) Bereitstellen der Vorrichtung (1), wobei der erste Behälter (2) mit einer ersten flüssigen oder pastenförmigen Komponente des PMMA-Knochenzements gefüllt ist und der zumindest eine zweite Behälter (4, 5) mit einer zweiten, vorzugsweise pulverförmigen oder pastenförmigen Komponente des PMMA-Knochenzements gefüllt ist;
B) Öffnen des zumindest einen zweiten Behälters (4, 5) durch Vorschieben des Dosierkolbens (6, 24) und Austragen der zweiten Komponente aus dem zumindest einen zweiten Behälter (4, 5) in den ersten Behälter (2) durch weiteres Vortreiben des Dosierkolbens (6, 24), wobei die Volumenveränderung des Inhalts des ersten Behälters (2) beim Eintragen der zweiten Komponente in den ersten Behälter (2) durch eine Bewegung des zumindest einen Volumenausgleichselements (50) ausgeglichen wird, wobei der Dosierkolben (6, 24) oder die Dosierkolben (6, 24) nach vollständigem Einschieben durch eine Rastung (26, 28) irreversibel gegen den zumindest einen zweiten Behälter (4, 5) fixiert wird oder werden; und
C) Mischen der beiden Komponenten im ersten Behälter (2) durch Bewegen der Mischeinrichtung (36), wobei beim Bewegen der Mischeinrichtung (36) das mit der Mischeinrichtung (36) verbundene Austragsrohr (10) in den ersten Behälter (2) wiederholt hineingeschoben und herausgezogen wird, wobei die Volumenveränderung des Inhalts des ersten Behälters (2) beim Mischen durch eine Bewegung des zumindest einen Volumenausgleichselements (50) ausgeglichen wird.

13. Verfahren nach Anspruch 12, **dadurch gekennzeichnet, dass** nach Schritt C) ein Kern (30) aus dem Austragsrohr (10) entfernt wird und danach ein Schritt D) erfolgt, bei dem der gemischte Knochenzement durch Vortreiben des Austragskolbens (18) in dem ersten Behälter (2) durch das Austragsrohr (10) appliziert wird.

14. Verfahren nach Anspruch 12 oder 13, **dadurch gekennzeichnet, dass**
der Inhalt des ersten Behälters (2) gemischt wird, indem die mit dem Austragsrohr (10) verbunden Mischeinrichtung (36) durch hineinbewegen und herausbewegen des Austragsrohrs (10) in den ersten Behälter (2) in dem ersten Behälter (2) bewegt wird, wobei bevorzugt zusätzlich die Mischeinrichtung (36) durch Drehen des Austragsrohrs (10) im ersten Behälter (2) gedreht wird.

15. Verfahren nach einem der Ansprüche 12 bis 14, **dadurch gekennzeichnet, dass**
vor Schritt A) das Innere des ersten Behälters (2) und das Innere des zumindest einen zweiten Behälters (4, 5) entgast und sterilisiert wird, wobei bevorzugt dazu der Begrenzungskolben (52), der vordere Austragskolben (50) und/oder das Volumenausgleichselement (50) arretiert wird, und anschließend der erste Behälter (2) mit einer ersten Komponente des Knochenzements und der zumindest eine zweite Behälter (4, 5) mit einer zweiten Komponente des Knochenzements gefüllt wird, wobei bevorzugt dabei oder danach ein Antibiotikum oder ein Antibiotikagemisch in wenigstens einen zweiten Behälter (4, 5) gefüllt wird.

## Claims

1. Device (1) for storing, mixing, and applying polymethylmethacrylate bone cement, comprising a first container (2) for a first pasty component of the bone cement, a dispensing plunger (18) that is arranged such that it can be shifted in the first container (2) and serves for extruding the content of the first container (2) through a dispensing tube (10) opposite from the dispensing plunger (18), whereby the dispensing tube (10) is arranged such that it can be rotated and shifted in longitudinal direction through a feed-through in a side of the first container (2) opposite from the dispensing plunger (18), and a mixing facility (36) for mixing the content of the first container (2), whereby the mixing facility (36) is arranged in the first container (2) and is secured to the dispensing tube (10) such that the mixing facility (36) can be moved in the first container (2) by moving the dispensing tube (10) in order to mix the content of the first container (2), whereby at least one second container (4, 5) for at least one second component of the bone cement is arranged on the first container (2), whereby the internal space of the at least one second container (4, 5) is closed with respect to the internal space of the first container (2) by means of a closure (22), which is openable, and whereby the at least one second container (4, 5) is limited, on the side opposite from the closure (22), by a dosing plunger (6, 24), and whereby at least one limiting surface of the first container (2) is formed by a mobile volume compensation element (50), **characterised in that**
the at least one dosing plunger (6, 24) possesses at least one directional snap-in element (26) that can engage at least one opposite snap-in mechanism (28) on the outer surface of the second container (4, 5) in such a manner that, after snapping-in takes place, a backward motion of the dosing plunger (6, 24) out of the second container(s) (4, 5) is not feasible.

2. Device (1) according to claim 1, **characterised in that**
the closure (22) of the at least one second container (4, 5) is openable by exerting a pressure on the dosing plunger (6, 24) of said second container (4, 5) such that the first container (2) is then connected to said second container (4, 5).

3. Device (1) according to claim 1 or 2, **characterised in that**
the mixing facility (36) comprises at least two mixing vanes (36) that are arranged on the end of the dispensing tube (10) pointing into the inside of the first container (2) and that extend radially from the dispensing tube (10) outwards into the first container (2).

4. Device (1) according to claim 3, **characterised in that**
the axial height of the mixing vane (36) is larger than the maximum outer diameter of the closure (22), whereby the hollow space between the mixing vanes (36) is sufficient to accommodate at least the closure (22) or closures (22).

5. Device (1) according to any one of the preceding claims, **characterised in that** the device (1) comprises two second containers (4, 5), which both are limited on two opposite sides by one closure (22) each and one dosing plunger (6, 24) each for dispensing the content of the second containers (4, 5).

6. Device (1) according to any one of the preceding claims, **characterised in that** the at least one closure (22) is connected to a ring (40) by means of a deformable connection (38), in particular a flexible fin (38), whereby the ring (40) is arranged on the inside of the first container (2) such as to be mobile about the dispensing tube (10) such that the connection (38) is axially mobile on the dispensing tube (10) by means of the ring (40), or whereby the ring (40) is secured to a guide sleeve (46) which is arranged in the feed-through for the dispensing tube (10) of the first container (2) and which guides the dispensing tube (10).

7. Device (1) according to any one of the preceding claims, **characterised in that** the dispensing plunger (18) is lockable or is locked with respect to the first container (2), preferably on the end of the first container (2) opposite from the dispensing tube (10).

8. Device (1) according to any one of the preceding claims, **characterised in that** the volume compensation element (50) is implemented by means of the dispensing plunger (18), whereby the dispensing plunger (18) is designed to be made of two parts, in form of a front dispensing plunger (50) and a limiting plunger (52), which are supported in the first container (2) such as to be mobile with respect to each other, and the motion of the front dispensing plunger (50) out of the first container (2) is limited by the limiting plunger (52), whereby the limiting plunger (52) is lockable in the first container (2), whereby the limiting plunger (52) preferably comprises a snap-in mechanism (58) that can be detached from outside and engages an opposite snap-in mechanism on the first container (2).

9. Device (1) according to claim 8, **characterised in that**
a gas passage opening is provided in the limiting plunger (52) and/or a gas passage opening is provided between the limiting plunger (52) and the first container (2), whereby the gas passage opening(s) is or are well-suited for discharging a gas from and filling a gas in between the front dispensing plunger (50) and the limiting plunger (52).

10. Device (1) according to any one of the preceding claims, **characterised in that** at least one volume compensation element (50) is supported as in a bearing through an elastic spring (54) such as to be mobile with respect to the first container (2), whereby the spring (54) pushes the volume compensation element (50) in the direction of the internal space of the first container (2).

11. Device (1) according to any one of the preceding claims, **characterised in that** at least one porous gas-permeable sealing ring is arranged on the external surface of the at least one dosing plunger (6, 24) such that a gas exchange between the space formed by the inner walls of the at least one second container (4, 5), the closure (22), and the dosing plunger (6, 24), and the surrounding atmosphere can take place.

12. Method for producing a polymethylmethacrylate bone cement using a device (1) according to any one of the preceding claims, having the steps of:
A) providing the device (1), whereby the first container (2) is filled with a first liquid or pasty component of the PMMA bone cement and the at least one second container (4, 5) is filled with a second component of the PMMA bone cement, which preferably is powdery or pasty;
B) opening the at least one second container (4, 5) by pushing the dosing plunger (6, 24) forward and dispensing the second component from the at least one second container (4, 5) into the first container (2) by further pushing the dosing plunger (6, 24) forward, whereby the change of volume of the content of the first container (2) upon the introduction of the second component into the first container (2) is compensated for through a motion of the at least one volume compensation element (50), whereby the dosing plunger (6, 24) or dosing plungers (6, 24), after being pushed in completely, is or are affixed irreversibly against the at least one second container (4, 5) by means of a snap-in mechanism (26, 28); and
C) mixing the two components in the first container (2) through moving the mixing facility (36), whereby moving the mixing facility (36) is associated with the dispensing tube (10) connected to the mixing facility (36) being pushed into and pulled out of the first container (2) repeatedly, whereby the change of volume of the content of the first container (2) during mixing is compensated for through a motion of the at least one volume compensation element (50).

13. Method according to claim 12, **characterised in that**
a core (30) is removed from the dispensing tube (10) after step C), and then a step D) takes place, in which the mixed bone cement is applied by pushing the dispensing plunger (18) forward in the first container (2) through the dispensing tube (10).

14. Method according to claim 12 or 13, **characterised in that**
the content of the first container (2) is mixed by moving the mixing facility (36), which is connected to the dispensing tube (10), in the first container (2) by moving the dispensing tube (10) into and out of the first container (2), whereby, in addition, the mixing facility (36) preferably is being rotated in the first container (2) by rotating the dispensing tube (10).

15. Method according to any one of the claims 12 to 14, **characterised in that** the interior of the first container (2) and the interior of the at least one second container (4, 5) are de-gassed and sterilised before step A) takes place, whereby it is preferred for this purpose to lock the limiting plunger (52), the front dispensing plunger (50) and/or the volume compensation element (50), and subsequently the first container (2) is filled with a first component of the bone cement and the at least one second container (4, 5) is filled with a second component of the bone cement, whereby it is preferred, concurrently or subsequently, to fill an antibiotic or a mixture of antibiotics into at least one second container (4, 5).

## Revendications

1. Dispositif (1) pour le stockage, le mélange et l'application de ciment osseux de polyméthacrylate de méthyle présentant un premier récipient (2) pour un premier composant de forme pâteuse du ciment osseux, un piston d'évacuation (18) disposé en pouvant se déplacer dans le premier récipient (2) pour l'expulsion sous pression du contenu du premier récipient (2) par un tube d'évacuation (10) situé en face du piston d'évacuation (18), où le tube d'évacuation (10) est disposé en pouvant être mis en rotation et déplacé dans la direction longitudinale par un passage dans un côté du premier récipient (2) se situant en face du piston d'évacuation (18), et un dispositif de mélange (36) pour le mélange du contenu du premier récipient (2), où le dispositif de mélange (36) est disposé dans le premier récipient (2) et est fixé sur le tube d'évacuation (10), de sorte que le dispositif de mélange (36) peut être déplacé dans le premier récipient (2) par le déplacement du tube d'évacuation (10) pour le mélange du contenu du premier récipient (2), où au moins un deuxième récipient (4, 5) pour au moins un deuxième composant du ciment osseux est disposé au niveau du premier récipient (2), où l'espace interne de l'au moins un deuxième récipient (4, 5) est fermé vis-à-vis de l'espace interne du premier récipient (2) par un couvercle (22) à ouvrir et l'au moins un deuxième récipient (4, 5) est délimité par un piston de dosage (6, 24) sur le côté en face du couvercle (22) et où au moins une surface de délimitation du premier récipient (2) est formé par un élément de compensation de volume (50) mobile, **caractérisé en ce que**
l'au moins un piston de dosage (6, 24) possède au moins un élément de verrouillage (26) orienté qui peut se mettre en prise sur le côté externe du deuxième récipient (4, 5) sur l'au moins un verrouillage complémentaire (28) de telle manière qu'après un verrouillage réussi, un mouvement de recul du piston de dosage (6, 24) à partir ou dans le deuxième récipient (4, 5) vers l'extérieur n'est pas possible.

2. Dispositif (1) selon la revendication 1, **caractérisé en ce que** le couvercle (22) de l'au moins un deuxième récipient (4, 5) peut s'ouvrir par l'exercice d'une pression sur le piston de dosage (6, 24) de ce deuxième récipient (4, 5), de sorte qu'alors le premier récipient (2) est relié avec ce deuxième récipient (4, 5).

3. Dispositif (1) selon la revendication 1 ou 2, **caractérisé en ce que** le dispositif de mélange (36) présente au moins deux pales de mélange (36) qui sont disposées à l'extrémité du tube d'évacuation (10) orientée vers l'intérieur du premier récipient (2) et qui s'étendent radialement à partir du tube d'évacuation (10) vers l'extérieur dans le premier récipient (2).

4. Dispositif (1) selon la revendication 3, **caractérisé en ce que**
la hauteur axiale des pales de mélange (36) est supérieure au diamètre extérieur maximal du couvercle (22), où l'espace creux entre les pales (36) est suffisant au moins pour la réception du couvercle (22) ou des couvercles (22).

5. Dispositif selon l'une des revendications précédentes, **caractérisé en ce que**
le dispositif (1) présente deux deuxièmes récipients (4, 5) qui sont tous les deux délimités sur deux côtés opposés par respectivement un couvercle (22) et respectivement un piston de dosage (6, 24) pour l'évacuation du contenu du deuxième récipient (4, 5).

6. Dispositif selon l'une des revendications précédentes, **caractérisé en ce que**
l'au moins un couvercle (22) est relié avec une bague (40) par l'intermédiaire d'une liaison (38) déformable, notamment une tige (38) souple, où la bague (40) est disposée mobile autour du tube d'évacuation à l'intérieur du premier récipient (2), de sorte que la liaison (38) avec la bague (40) peut se déplacer sur le tube d'évacuation (10), ou la bague (40) est fixée à une gaine de guidage (48) qui est disposée dans le passage pour le tube d'évacuation (10) du premier récipient (2) et qui guide le tube d'extraction (10).

7. Dispositif selon l'une des revendications précédentes, **caractérisé en ce que**
le piston d'évacuation (18) peut être verrouillé ou est verrouillé contre le premier récipient (2), de préférence, peut être verrouillé ou est verrouillé à l'extrémité du premier récipient (2) opposée au tube d'évacuation (10).

8. Dispositif selon l'une des revendications précédentes, **caractérisé en ce que** l'élément de compensation de volume (50) est concrétisé par le piston d'évacuation (18), où le piston d'évacuation (18) est conçu en deux parties par un piston d'évacuation frontal (50) et un piston de limitation (52), qui sont logés mobiles l'un à l'encontre de l'autre dans le premier récipient (2), et le mouvement du piston d'évacuation frontal (50) à partir du premier récipient (2) est limité par le piston de limitation (52), où le piston de limitation (52) peut être verrouillé dans le premier récipient (2), où, de préférence, le piston de limitation (52) présente un système de verrouillage (58) pouvant être déclenché de l'extérieur, qui se met en prise dans un système de verrouillage complémentaire sur le premier récipient (2).

9. Dispositif selon la revendication 8, **caractérisé en ce que**
dans le piston de limitation (52), il est prévu un orifice de passage de gaz, et/ou un orifice de passage de gaz est prévu entre le piston de limitation (52) et le premier récipient (2), qui est approprié ou qui sont appropriés pour l'évacuation et le remplissage d'un gaz entre le piston d'évacuation frontal (50) et le piston de limitation (52).

10. Dispositif selon l'une des revendications précédentes, **caractérisé en ce**
**qu'**au moins un élément de compensation de volume (50) est logé mobile contre le premier récipient (2) par l'intermédiaire d'un ressort (54) élastique, où le ressort (54) presse l'élément de compensation de volume (50) en direction de l'espace intérieur du premier récipient (2).

11. Dispositif selon l'une des revendications précédentes, **caractérisé en ce qu'**au niveau du côté extérieur de l'au moins un piston de dosage (6, 24) au moins une bague d'étanchéité poreuse, perméable aux gaz est disposée de sorte qu'un échange de gaz est possible à partir de l'espace formé par les parois intérieures de l'au moins un deuxième récipient (4, 5), le couvercle (22) et le piston de dosage (6, 24) et l'atmosphère environnante.

12. Procédé de fabrication d'un ciment osseux de polyméthacrylate de méthyle avec un dispositif (1) selon l'une des revendications précédentes, avec les étapes :
A) de mise à disposition du dispositif (1), où le premier récipient (2) est rempli avec un premier composant liquide ou de forme pâteuse du ciment osseux de PMMA et l'au moins un deuxième récipient (4, 5) est rempli avec un deuxième composant de préférence sous forme de poudre ou sous forme de pâte du ciment osseux de PMMA ;
B) d'ouverture de l'au moins un deuxième récipient (4, 5) par une avancée du piston de dosage (6, 24) et d'évacuation du deuxième composant à partir de l'au moins un deuxième récipient (4, 5) dans le premier récipient (2) par une nouvelle avancée du piston de dosage (6, 24), où la modification de volume du contenu du premier récipient (2) lors de l'introduction du deuxième composant dans le premier récipient (2) est compensée par un mouvement de l'au moins un élément de compensation de volume (50), où le piston de dosage (6, 24), ou les pistons de dosage (6, 24), est fixé ou sont fixés de manière irréversible contre l'au moins un deuxième récipient (4, 5) après l'introduction complète par le système de verrouillage (26, 28) ; et
C) de mélange des deux composants dans le premier récipient (2) par un déplacement du dispositif de mélange (36), où, lors du déplacement du dispositif de mélange (36) le tube d'évacuation (10) relié avec le dispositif de mélange (36) est introduit et retiré de manière répétée du premier récipient (2), où la modification de volume du contenu du premier récipient (2) est compensée par un mouvement de l'au moins un élément de compensation de volume (50) lors du mélange.

13. Procédé selon la revendication 12, **caractérisé en ce**
**qu'**après l'étape C), une partie centrale (30) est extraite du tube d'évacuation (10) et qu'ensuite l'étape D) a lieu, dans laquelle le ciment osseux mélangé est appliqué par une avancée du tube d'évacuation (18) dans le premier récipient (2) par le tube d'évacuation (10).

14. Procédé selon la revendication 12 ou 13, **caractérisé en ce que**
le contenu du premier récipient (2) est mélangé, **en ce que** le dispositif de mélange (36) relié avec le tube d'évacuation (10) est entré et sorti du tube d'évacuation (10) dans le premier récipient (2) dans le premier récipient (2), où, de préférence, le dispositif de mélange (36) est en outre mis en rotation par la mise en rotation du tube d'évacuation (10) dans le premier récipient (2).

15. Procédé selon l'une des revendications 12 à 14, **caractérisé en ce**
**qu'**avant l'étape A), l'intérieur du premier récipient (2) et l'intérieur de l'au moins un deuxième récipient (4, 5) est dégazé et stérilisé, où de préférence, de manière complémentaire, le piston de limitation (52), le piston d'évacuation (50) frontal et/ou l'élément de compensation de volume (50), est verrouillé, et ensuite le premier récipient (2) est rempli avec un premier composant du ciment osseux et l'au moins un deuxième récipient (4, 5) est rempli avec un deuxième composant du ciment osseux, où, de préférence, ce faisant ou après, un antibiotique ou un mélange d'antibiotiques est rempli dans au moins un deuxième récipient (4, 5).
